# EUROPEAN PATENT APPLICATION

(11) **EP 4 660 202 A1**
(43) Date of publication of application: **10.12.2025**
(21) Application number: 24744874.9
(22) Date of filing: 17.01.2024
(51) Int. Cl.: C07K 16/28, C07K 14/725, A61K 47/68, A61P 19/00, A61P 3/00, A61K 39/00

(54) **REGULATORY T CELL SURFACE ANTIGEN, AND ANTIBODY THAT SPECIFICALLY BINDS THERETO**

(30) Priority: 17.01.2023 KR 20230006547
(71) Applicant: Good T Cells, Inc., Seoul 03929 (KR)
(72) Inventor: KIM, Jung Ho, Seoul 04136 (KR); KIM, Beom Seok, Seoul 03488 (KR)
(74) Representative: Tergau & Walkenhorst Intellectual Property GmbH
(86) International application number: PCT/KR2024/000837
(87) International publication number: WO 2024/155094

(57) **Abstract**

The present invention relates to an epitope of Lrig1 (leucine-rich and immunoglobulin-like domains protein 1) protein, which is an antigen present on the surface of regulatory T cells, and to an antibody or an antigen-binding fragment that specifically binds thereto.

The present invention also provides compositions for diagnosis of metabolic diseases, diagnostic kits, methods of providing information for diagnosis, or diagnostic devices comprising as an active ingredient an antibody or antigen-binding fragment capable of specifically binding to Lrig-1 (leucine-rich and immunoglobulin-like domains 1) protein present on the surface of regulatory T cells or adipocytes.

## Description

### Technical Field

The present invention relates to antigens present on the surface of regulatory T cells, the Lrig-1 (leucine-rich and immunoglobulin-like domains 1) protein, and antibodies or antigen-binding fragments that specifically bind thereto.

### Background Art

One of the most important traits in all normal individuals is their ability to recognize and eliminate non-self antigens while not reacting harmfully to self-antigenic substances that make up the self. As such, the non-response of a living body to self-antigens is called immunologic unresponsiveness or tolerance. Self-tolerance occurs either by elimination of lymphocytes that may have specific receptors for self-antigens or by inactivation of self-reactive lymphocytes after contact with self-antigens. Abnormalities in the induction or maintenance of self-tolerance lead to immune responses against self-antigens, which may result in disorders called autoimmune diseases.

For the treatment of the autoimmune disease, the concept of suppressor T cells with the possibility of presence of T cells capable of controlling and suppressing the effector function of conventional T cells was first introduced and proposed by Gershon in the early 1970s. Since then, research has been conducted to elucidate the biological properties and functions of regulatory T cells in many fields of immunology.

Accordingly, the regulatory T cells (Treg) play an important role in naturally preventing the occurrence of excessive inflammation and immune responses, but it has been reported that, when autoimmune diseases and chronic inflammatory diseases occur, the function and number of regulatory T cells remarkably decrease. Thus, for patients with immune diseases and inflammatory diseases, it is important that regulatory T cells are generated at normal levels, which can be one of methods for treatment of these diseases.

Until now, studies on genes and proteins present specifically in regulatory T cells have been conducted, suggesting that substances such as CD25, CTLA4, CD62L, CD38, CD103, GITR, and CD45RB may correspond to marker substances. However, genes or proteins that can target regulatory T cells alone have not yet been reported.

Meanwhile, there are three hypervariable regions, called complementarity determining regions (hereinafter referred to as "CDRs"), and four framework regions. The CDRs mainly serve to bind to an antigenic determinant (epitope) of an antigen. The CDRs of each chain are typically referred to as CDR1, CDR2, and CDR3, numbered sequentially starting from the N-terminus, and are also identified by the chain in which the particular CDR is located.

### DISCLOSURE

### Technical Problem

An object of the present invention is to provide pharmaceutical compositions for the prevention or treatment of metabolic diseases comprising as an active ingredient an antibody or antigen-binding fragment capable of specifically binding to Lrig-1 (leucine-rich and immunoglobulin-like domains 1) proteins present on the surface of regulatory T cells (Treg cells).

Another object of the present invention is to provide an antibody-drug conjugate (ADC) in which a drug for preventing or treating metabolic diseases is conjugated to the antibody according to the present invention.

Still Another object of the present invention is to provide pharmaceutical compositions for the prevention or treatment of various diseases, for example metabolic diseases, comprising an antibody-drug conjugate as an active ingredient.

An object of the present invention is to provide a composition for the diagnosis of metabolic diseases, a diagnostic kit, a method of providing information for diagnosis, or a diagnostic device comprising as an active ingredient an antibody or antigen-binding fragment capable of binding specifically to the Lrig-1 (leucine-rich and immunoglobulin-like domains 1) protein present on the surface of a regulatory T cell (Treg cell).

However, objects to be achieved by the present invention are not limited to the objects mentioned above, and other objects not mentioned above will be clearly understood by those of ordinary skill in the art from the following description.

### Technical Solution

The present invention is directed to an epitope of Lrig-1 (leucine-rich and immunoglobulin-like domains 1) protein or an antibody or antigen-binding fragment that binds specifically to the epitope.

In the present invention, the "epitope" refers to a sequence of amino acids in an antigen wherein the amino acids (or a subset thereof) in the sequence are specifically recognized by an antibody or binding fragment, for example an antibody or binding fragment described herein. An epitope may comprise one or more antigenic determinants. For example, an antibody generated against an isolated peptide corresponding to a conformational epitope recognizes part or all of said epitope sequence.

In the present invention, the "Lrig-1 protein" is a transmembrane protein present on the surfaces of regulatory T cells, and is composed of leucine-rich repeats (LRRs) and three immunoglobulin-like domains on the extracellular or lumen side, a cell transmembrane sequence, and a cytoplasmic tail region. The LRIG gene family includes LRIG1, LRIG2, and LRIG3, and the amino acids constituting each member of the family are highly conserved. The LRIG1 gene is highly expressed in normal skin, and can be expressed in basal and hair follicle cells to regulate proliferation of epithelial stem cells. Thus, the LRIG1 gene plays an important role in maintaining homeostasis of the epidermis, and its absence may lead to psoriasis or skin cancer. It has been reported that a deletion of chromosome region 3p14.3 in which LRIG1 is located is likely to develop into cancer cells. In fact, it was found that expression of LRIG1 greatly decreased in renal cell carcinoma and cutaneous squamous cell carcinoma. In recent years, it has also been found that the Lrig-1 protein is expressed in only about 20 to 30% of cancers. Meanwhile, for the purpose of the present invention, the Lrig-1 protein may be a protein derived from mammals or mice, without being limited thereto.

As an example of the present invention, the Lrig-1 protein may be a Lrig-1 protein derived from mammals, including primates such as humans and monkeys, rodents such as mice and rats, or the like.

As an example of the present invention, the Lrig-1 protein may be a human-derived Lrig-1 protein represented by SEQ ID NO: 1, which may be encoded by the nucleic acid sequence represented by SEQ ID NO: 2, without being limited thereto.

As another example of the present invention, the Lrig-1 protein may be a mouse-derived Lrig-1 protein represented by SEQ ID NO: 3, which may be encoded by the nucleic acid sequence represented by SEQ ID NO: 4, without being limited thereto.

As another example of the present invention, the Lrig-1 protein may be an extracellular domain of the Lrig-1 protein, without being limited thereto.

The Lrig-1 extracellular domain in the present invention may be an extracellular domain of Lrig-1 protein derived from mammals including primates such as humans and monkeys, rodents such as mice and rats, or the like. For the purpose of the present invention, the extracellular protein of the Lrig-1 protein may be an extracellular domain of Lrig-1 protein derived from a human or mouse, without being limited thereto.

As an example of the present invention, the extracellular domain of the Lrig-1 protein may be represented by SEQ ID NO: 5, which corresponds to amino acid positions 35 to 794 in the amino acid sequence of the human-derived Lrig-1 protein, without being limited thereto.

As another example of the present invention, the extracellular domain of the Lrig-1 protein may be represented by SEQ ID NO: 6, which corresponds to amino acid positions 35 to 794 in the amino acid sequence of the mouse-derived Lrig-1 protein, without being limited thereto.

Hereinafter, the present invention will be described in more detail.

In accordance with one embodiment of the present invention, there is provided an epitope of the Lrig-1 protein, the epitope comprising at least one polypeptide selected from the group consisting of polypeptides consisting of the amino acid sequence represented by SEQ ID NOs: 35 to 45.

As an example of the present invention, the epitope of the Lirg-1 protein may be an epitope comprising at least one polypeptide selected from the group consisting of polypeptides consisting of the amino acid sequence represented by SEQ ID NOs: 35 to 40, without being limited thereto.

As another example of the present invention, the epitope of the Lirg-1 protein may be an epitope comprising at least one polypeptide selected from the group consisting of polypeptides consisting of the amino acid sequence represented by SEQ ID NOs: 41 to 45, without being limited thereto.

According to one embodiment of the present invention, the epitope of the Lrig-1 protein may be a region corresponding to the amino acid sequence at positions 62 to 101, or 88 to 92, or 90 to 101, or 111 to 134, or 452 to 476, or 472 to 480, or 542 to 553 of the Lrig-1 protein.

According to one embodiment of the present invention, the epitope of the Lrig-1 protein may be a region corresponding to the amino acid sequence at positions 95 to 101, or 472 to 476 of the Lrig-1 protein.

The epitope of the present invention may be a conformational epitope.

The "conformational epitope" of the present invention consists of a discontinuous amino acid sequence, unlike a one-dimensional linear epitope consisting of a continuous sequence. This conformational epitope reacts with the three-dimensional structure of the antigen-binding site of an antibody.

Another embodiment of the present invention provides a binding molecule that binds specifically to an epitope of the present invention.

The "binding molecule" of the present invention may be either an antibody or an antigen-binding fragment, without being limited thereto.

In the present invention, the antibody is a full-length antibody or a part of the antibody, has the ability to bind to the Lrig-1 protein, and includes any antibody fragment that binds to the Lrig-1 antigen determining region in a manner competitive with the binding molecule of the present invention.

In the present invention, the term "antibody" refers to a protein molecule which serves as a receptor that specifically recognizes an antigen, including an immunoglobulin molecule that is immunologically reactive with a particular antigen. For the purpose of the present invention, the antigen may be Lrig-1 protein present on the surfaces of regulatory T cells. Preferably, the antibody may be one that specifically recognizes the leucine-rich region or immunoglobulin-like domain of the Lrig-1 protein, without being limited thereto.

In the present invention, the "immunoglobulin" has a heavy chain and a light chain, and each of the heavy chain and the light chain comprises a constant region and a variable region. The variable region of each of the light chain and the heavy chain contains three hypervariable regions, called complementarity determining regions (hereinafter referred to as "CDRs"), and four framework regions. The CDRs primarily serve to bind to an antigenic determinant (epitope) of an antigen. The CDRs of each chain are typically referred to as CDR1, CDR2, and CDR3, numbered sequentially starting from the N-terminus, and are also typically identified by the chain in which the particular CDR is located.

In the present invention, the antibody or antigen-binding fragment thereof provided in the present invention is a chimeric antibody or fragment comprising heavy chain and light chain CDRs selected from the CDRs provided in the present invention, or conservative variants of the CDRs provided in the present invention.

For the purpose of the present invention, "variants" of an amino acid sequence comprise amino acid insertion variants, amino acid addition variants, amino acid deletion variants and/or amino acid substitution variants. Amino acid deletion variants that comprise the deletion at the N-terminal and/or C-terminal end of the protein are also called N-terminal and/or C-terminal truncation variants.

Amino acid insertion variants comprise insertions of single or two or more amino acids in a particular amino acid sequence. In the case of amino acid sequence variants having an insertion, one or more amino acid residues are inserted into a particular site in an amino acid sequence, but random insertion with appropriate screening of the resulting product is also possible.

Amino acid addition variants comprise amino- and/or carboxy-terminal fusions of one or more amino acids, such as 1, 2, 3, 5, 10, 20, 30, 50, or more amino acids.

Amino acid deletion variants are characterized by the removal of one or more amino acids from the sequence, such as by removal of 1, 2, 3, 5, 10, 20, 30, 50, or more amino acids. The deletions may be in any position of the protein.

Amino acid substitution variants are characterized by at least one residue in the sequence being removed and another residue being inserted in its place. Preference is given to modifications being in positions in the amino acid sequence which are not conserved between homologous proteins or peptides and/or to replacing amino acids with other ones having similar properties. Preferably, amino acid changes in protein variants are conservative amino acid changes, i.e., substitutions of similarly charged or uncharged amino acids. A conservative amino acid change involves substitution of one of a family of amino acids which are related in their side chains. Naturally occurring amino acids are generally divided into four families: acidic (aspartate, glutamate), basic (lysine, arginine, histidine), non-polar (alanine, valine, leucine, isoleucine, proline, phenylalanine, methionine, tryptophan), and uncharged polar (glycine, asparagine, glutamine, cysteine, serine, threonine, tyrosine) amino acids. Phenylalanine, tryptophan, and tyrosine are sometimes classified jointly as aromatic amino acids.

Preferably, the degree of similarity, preferably identity between a given amino acid sequence and an amino acid sequence which is a variant of said given amino acid sequence will be at least about 60%, 65%, 70%, 80%, 81%, 82%, 83%, 84%, 84%, 85%, 86%, 87%, 88%, 89%, 90%, 91%, 92%, 93%, 94%, 95%, 96%, 97%, 98%, or 99%. The degree of similarity or identity is given preferably for an amino acid region which is at least about 10%, at least about 20%, at least about 30%, at least about 40%, at least about 50%, at least about 60%, at least about 70%, at least about 80%, at least about 90% or about 100% of the entire length of the reference amino acid sequence. For example, if the reference amino acid sequence consists of 200 amino acids, the degree of similarity or identity is given preferably for at least about 20, at least about 40, at least about 60, at least about 80, at least about 100, at least about 120, at least about 140, at least about 160, at least about 180, or about 200 amino acids, preferably continuous amino acids. In preferred embodiments, the degree of similarity or identity is given for the entire length of the reference amino acid sequence. The alignment for determining sequence similarity, preferably sequence identity can be done with tools known in the art, preferably using the best sequence alignment, for example, using Align, using standard settings, preferably EMBOSS::needle, Matrix: Blosum62, Gap Open 10.0, Gap Extend 0.5.

"Sequence similarity" indicates the percentage of amino acids that either are identical or that represent conservative amino acid substitutions. "Sequence identity" between two amino acid sequences indicates the percentage of amino acids that are identical between the sequences.

The term "percentage identity" is intended to denote a percentage of amino acid residues which are identical between the two sequences to be compared, obtained after the best alignment, this percentage being purely statistical and the differences between the two sequences being distributed randomly and over their entire length. Sequence comparisons between two amino acid sequences are conventionally carried out by comparing these sequences after having aligned them optimally, said comparison being carried out by segment or by "window of comparison" in order to identify and compare local regions of sequence similarity. The optimal alignment of the sequences for comparison may be produced, besides manually, by means of the local homology algorithm, or by means of a similarity search method, or by means of computer programs which use these algorithms (GAP, BESTFIT, FASTA, BLAST P, BLAST N and TFASTA in Wisconsin Genetics Software Package, Genetics Computer Group, 575 Science Drive, Madison, Wisconsin, USA).

The percentage identity is calculated by determining the number of identical positions between the two sequences being compared, dividing this number by the number of positions compared and multiplying the result obtained by 100 so as to obtain the percentage identity between these two sequences.

Homologous amino acid sequences exhibit, according to the present invention, at least 40%, in particular at least 50%, at least 60%, at least 70%, at least 80%, at least 90% and preferably at least 95%, at least 98 or at least 99% identity of the amino acid residues.

The amino acid sequence variants described herein may readily be prepared by those skilled in the art, for example, by recombinant DNA manipulation. The manipulation of DNA sequences for preparing peptides or proteins having substitutions, additions, insertions or deletions, is well known. Furthermore, the peptides and amino acid variants described herein may be readily prepared with the aid of known peptide synthesis techniques, for example, by solid phase synthesis and similar methods.

In the present invention, the antibody or antigen binding fragment thereof provided in the present invention is a humanized antibody or fragment comprising heavy and light chain CDRs selected from the CDRs provided in the present invention, or conservative variants of the CDRs provided in the present invention.

In the present invention, the antibody or antigen binding fragment thereof provided in the present invention comprises a light chain and/or a heavy chain comprising a sequence provided in the present invention, or a conservative variant thereof. In one embodiment, the conservative variants have at least 80%, at least 85%, at least 90%, at least 95%, at least 96%, at least 97%, at least 98% or at least 99% homology to the reference sequence provided in the present invention. In one embodiment, the conservative variants comprise 1, 2, 3, 4, 5, 6, 7, 8, 9, 10, or more amino acid substitutions, insertions, or deletions.

In the present invention, the antibodies provided in the present invention, which bind specifically to the Lrig-1 protein comprise the sequences provided herein or conservative variants thereof. "Conservative variants," as used herein, include conservative amino acid substitutions, insertions, or deletions. A person skilled in the art will recognize that a conservative amino acid substitution is a substitution of one amino acid with another amino acid that has similar structural or chemical properties, such as a similar side chain, and a conservative amino acid substitution results in a sequence that retains the biological activity of the reference sequence. Exemplary conservative substitutions are well known in the art.

In the present invention, the "full-length antibody" has a structure having two full-length light chains and two full-length heavy chains, wherein each of the light chains is linked to the heavy chain via a disulfide bond. Examples of the full-length antibody include IgA, IgD, IgE, IgM, and IgG. Subtypes of the IgG include IgG1, IgG2, IgG3, and IgG4.

In addition, in the present invention, the "antigen-binding fragment" refers to a fragment having an antigen-binding function. Examples of the antigen-binding fragment include: (1) a Fab fragment consisting of a light chain variable region (VL), a heavy chain variable region (VH), a light chain constant region (CL), and a heavy chain constant region 1 (CH1); (2) a Fd fragment consisting of VH and CH1 domains; (3) a Fv fragment consisting of VL and VH domains of a single antibody; (4) a dAb fragment consisting of a VH domain; (5) an isolated CDR region; (6) a F(ab')₂ fragment which is a bivalent fragment including two linked Fab fragments; (7) a single-chain Fv molecule (scFv) in which a VH domain and a VL domain are linked together by a peptide linker that allows the two domains to associate to form an antigen-binding site; (8) a bispecific single-chain Fv dimer; and (9) a diabody which is a multivalent or multispecific fragment constructed by gene fusion. The antigen-binding fragment may be obtained as a Fab or F(ab')₂ fragment when a proteolytic enzyme, for example, papain or pepsin is used, and the antigen-binding fragment may be produced through a genetic recombination technique.

In addition, in the present invention, the antibody or a fragment thereof may be, without limitation, a monoclonal antibody, a polyclonal antibody, a chimeric antibody, a humanized antibody, a human antibody, a bivalent-bispecific molecule, a minibody, a domain antibody, a bispecific antibody, an antibody mimetic, a unibody, a diabody, a triabody, a tetrabody, or a fragment thereof, without being limited thereto.

In the present invention, the "chimeric antibody" is an antibody which is obtained by recombination of a variable region of a mouse antibody and a constant region of a human antibody, and has a greatly improved immune response compared to the mouse antibody.

In addition, in the present invention, the "humanized antibody" refers to an antibody obtained by modifying the protein sequence of an antibody of non-human species origin so that the protein sequence is similar to an antibody variant naturally produced in humans. For example, the humanized antibody may be produced by recombining mouse-derived CDRs with a human antibody-derived FR to obtain a humanized variable region, and recombining the humanized variable region with a constant region of a desired human antibody.

In the present invention, the binding molecule may be provided as a bispecific antibody or bispecific antigen-binding fragment which is capable of binding to Lrig-1 protein and also binding to another protein.

In the present invention, the bispecific antibody and the bispecific antigen-binding fragment may comprise the binding molecule according to the present invention. As an example of the present invention, the bispecific antibody and the bispecific antigen-binding fragment comprise an antigen-binding domain capable of binding to Lrig-1 protein, wherein the antigen-binding domain capable of binding to Lrig-1 protein may comprise or consist of the binding molecule according to the present invention.

The bispecific antibody and bispecific antigen-binding fragment provided in the present invention comprise an antigen-binding domain, which is a binding molecule capable of binding to Lrig-1 protein according to the present invention, and an antigen-binding domain capable of binding to another target protein. Here, the antigen-binding domain capable of binding to another target protein may be an antigen-binding domain capable of binding to a protein other than Lrig-1 protein, for example, but not limited to, PD-1 or a cell surface receptor. However, the antigen-binding domain is not limited thereto.

The bispecific antibody and the bispecific antigen-binding fragment according to the present invention may be provided in any suitable format, for example, that described in the literature, which is incorporated herein by reference in its entirety. For example, the bispecific antibody or the bispecific antigen-binding fragment may be a bispecific antibody conjugate (for example, IgG2, F(ab')2, or CovX-body), a bispecific IgG or IgG-like molecule (for example, IgG, scFv4-Ig, IgG-scFv, scFv-IgG, DVD-Ig, IgG-sVD, sVD-IgG, or 2 in 1-IgG, mAb2, or Tandemab common LC), an asymmetric bispecific IgG or IgG-like molecule (for example, kih IgG, kih IgG common LC, CrossMab, kih IgG-scFab, mAb-Fv, charge pair, or SEED-body), a small bispecific antibody molecule (for example, diabody (Db), dsDb, DART, scDb, tandAb, tandem scFv (taFv), tandem dAb/VHH, triple body, triple head, Fab-scFv, or F(ab')2-scFv2), a bispecific Fc and CH3 fusion protein (for example, taFv-Fc, di-diabody, scDb-CH3, scFv-Fc-scFv, HCAb-VHH, scFv-kih-Fc, or scFv-kih-CH3), or a bispecific fusion protein (for example, scFv2-albumin, scDb-albumin, taFv-toxin, DNL-Fab3, DNL-Fab4-IgG, DNL-Fab4-IgG-cytokine 2). The bispecific antibody and bispecific antigen-binding fragment according to the present invention may be designed and produced by those skilled in the art.

In the present invention, a method for producing the bispecific antibody comprises chemical crosslinking of an antibody or antibody fragment with a reducing disulfide or non-reducing thioether bond. For example, N-succinimidyl-3-(-2-pyridyldithio)-propionate (SPDP) may be used, for example, for chemically crosslinking an Fab fragment through an SH-group at the hinge region, to generate a disulfide-linked bispecific F(ab)2 heterodimer.

In addition, an alternative method for producing the bispecific antibody according to the present invention comprises fusing an antibody-producing hybridoma with, for example, polyethylene glycol, to produce quadroma cells capable of secreting bispecific antibodies.

The bispecific antibody and bispecific antigen-binding fragment according to the invention may also be, for example, recombinantly produced by expression from a nucleic acid construct that encodes a polypeptide for an antigen-binding molecule.

For example, a DNA construct that contains a sequence encoding light and heavy chain variable domains for two antigen-binding domains (that is, light and heavy chain variable domains for an antigen-binding domain capable of binding to PD-1, and light and heavy chain variable domains for an antigen-binding domain capable of binding to another target protein), and a sequence encoding a suitable linker or dimerization domain between the antigen-binding domains may be prepared by molecular cloning techniques. Subsequently, a recombinant bispecific antibody may be produced by expression of the construct (for example, *in vitro*) in a suitable host cell (for example, a mammalian host cell), and then the expressed recombinant bispecific antibody may be optionally purified.

Antibodies may be produced by an affinity maturation process in which a modified antibody with improved affinity for an antigen as compared with an unmodified parent antibody is produced. An affinity-matured antibody may be produced by a procedure known in the art.

In addition, the binding molecule provided in the present invention may include a variant of the amino acid sequence as long as the variant can specifically bind to Lrig-1 protein. For example, in order to improve binding affinity and/or other biological properties of an antibody, modifications may be made to an amino acid sequence of the antibody. Such modifications include, for example, deletions, insertions, and/or substitutions of amino acid sequence residues of the antibody.

Such amino acid variations are made based on relative similarity of amino acid side chain substituents such as hydrophobicity, hydrophilicity, charge, and size. According to analysis on sizes, shapes, and types of amino acid side chain substituents, it can be seen that arginine, lysine, and histidine are all positively charged residues; alanine, glycine, and serine have similar sizes; and phenylalanine, tryptophan, and tyrosine have similar shapes. Thus, based on these considerations, it can be said that arginine, lysine, and histidine; alanine, glycine, and serine; and phenylalanine, tryptophan, and tyrosine are biologically functional equivalents.

To introduce variations, the hydropathic index of amino acids may be considered. Each amino acid has been assigned a hydropathic index on the basis of their hydrophobicity and charge characteristics, which are as follows: isoleucine (+4.5); valine (+4.2); leucine (+3.8); phenylalanine (+2.8); cysteine/cystine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamate (-3.5); glutamine (-3.5); aspartate (-3.5); asparagine (-3.5); lysine (-3.9); and arginine (-4.5). the hydropathic amino acid index is very important in conferring interactive biological function on a protein. It is known that certain amino acids may be substituted for other amino acids having a similar hydropathic index and still retain a similar biological activity. To introduce variations based on the hydropathic index, substitutions are made between amino acids that exhibit a hydropathic index difference of preferably within ±2, more preferably ±1, even more preferably ±0.5.

Meanwhile, it is also well known that substitution between amino acids having similar hydrophilicity values results in proteins having equivalent biological activity. As disclosed in U.S. Pat. No. 4,554,101, the following hydrophilicity values have been assigned to amino acid residues: arginine (+3.0); lysine (+3.0); aspartate (+3.0±1); glutamate (+3.0±1); serine (+0.3); asparagine (+0.2); glutamine (+0.2); glycine (0); threonine (-0.4); proline (-0.5±1); alanine (-0.5); histidine (-0.5); cysteine (-1.0); methionine (-1.3); valine (-1.5); leucine (-1.8); isoleucine (-1.8); tyrosine (-2.3); phenylalanine (-2.5); tryptophan (-3.4). To introduce variations based on the hydrophilicity value, substitutions may be made between amino acids that exhibit a hydrophilicity value difference of preferably within ±2, more preferably ±1, even more preferably ±0.5.

Amino acid exchanges in proteins, which do not generally alter the activity of molecules, are known in the art. The most commonly occurring exchanges are exchanges between amino acid residues Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, and Gln/Glu.

Considering the above-described variations having biologically equivalent activity, it is interpreted that the binding molecules of the present invention also include a sequence showing substantial identity with the sequence set forth in the Sequence Listing.

In the present invention, the term "substantial identity" means a sequence showing at least 61% homology, more preferably 70% homology, even more preferably 80% homology, most preferably 90% homology, as determined by aligning the sequence of the present invention with any other sequence to correspond to each other as much as possible and analyzing the aligned sequence using an algorithm commonly used in the art. Alignment methods for sequence comparison are known in the art. Various methods and algorithms for alignment are accessible from NCBI Basic Local Alignment Search Tool (BLAST), NCBI (National Center for Biological Information), etc., and may be used in conjunction with sequencing programs, such as blastp, blasm, blastx, tblastn and tblastx, on the Internet. BLAST is available from http://www.ncbi.nlm.nih.gov/BLAST/. Sequence homology comparison methods using this program can be identified online (http://www.ncbi.nim.nih.gov/BLAST/blast_help.html).

In the present invention, although the binding molecule, preferably the antibody, may be produced by a conventional method for producing an antibody, it may be produced by affinity maturation.

In the present invention, the "affinity maturation" refers to a process in which antibodies having increased affinity for an antigen are produced by activated B cells in the course of an immune response. For the purpose of the present invention, the affinity maturation allows antibodies or antibody fragments to be produced due to affinity maturation based on the principles of mutation and selection, in the same process as that occurring in nature.

In addition, in the present invention, the "monoclonal antibody" refers to an antibody molecule of a single molecular composition which is obtained from substantially the same antibody population, and exhibits single binding specificity and affinity for a particular epitope.

In the present invention, the "binding" or "specific binding" refers to the affinity of the antibody or antibody composition herein for an antigen. In antigen-antibody binding, the "specific binding" is distinguishable from non-specific background binding, typically in a case where a dissociation constant (Kd) is less than 1×10⁻⁵ M, less than 1×10⁻⁶ M, or less than 1×10⁻⁷ M. Specific binding can be detected by methods known in the art, such as ELISA, surface plasmon resonance (SPR), immunoprecipitation, and coprecipitation, which include an appropriate control that can distinguish between non-specific binding and specific binding.

The antibody or antigen-binding fragment of the present invention may exist as a multimer, such as a dimer, a trimer, a tetramer, or a pentamer, which includes at least part of antigen-binding capacity of a monomer. Such a multimer also includes a homomultimer or a heteromultimer. Antibody multimers contain a large number of antigen-binding sites, and thus have superior antigen-binding capacity as compared with monomers. Antibody multimers are also easily used to produce multifunctional (that is, bifunctional, trifunctional, tetrafunctional, or the like) antibodies.

In the present invention, the "multifunctional" refers to an antibody or antigen-binding fragment that has two or more activities or functions (for example, antigen-binding capacity, enzyme activity, and ligand- or receptor-binding capacity). For example, the antibody of the present invention may be bound to a polypeptide having enzymatic activity, such as luciferase, acetyltransferase, galactosidase, or the like. Multifunctional antibodies also include multivalent or multispecific (that is, bispecific, trispecific, or the like) forms of antibodies.

In addition, the antibody or antigen-binding fragment according to the present invention is capable of binding specifically to an epitope comprising a polypeptide represented by any one of the amino acid sequences of SEQ ID NOs: 35 to 45, which is present on regulatory T cells, thereby suppressing the function of the regulatory T cells and regulating the activity of effector T cells, thus preventing or treating various diseases, such as metabolic diseases.

In the present invention, "obesity" refers to the condition of excessive accumulation of body fat rather than simply being overweight, meaning that a person can be said to be obese if he or she has a high percentage of body fat even if he or she appears to be of normal weight. The most common way to determine obesity is through the body mass index (BMI), which is considered overweight if it is between 23 and 24.9, mild obesity if it is between 25 and 29.9, moderate obesity if it is between 30 and 34.9, and severe obesity if it is over 35. Obesity is caused by a combination of factors rather than a single cause, such as poor dietary habits, including westernized eating habits, decreased physical activity, emotional factors, and genetic factors, which in turn increase the risk of developing chronic diseases such as hyperlipidemia, diabetes, and hypertension.

In the present invention, "metabolic disease" refers to a condition or disease that is closely related to, or caused by, obesity, and may be one or more selected from the group consisting of fatty liver, type 2 diabetes, hyperlipidemia, cardiovascular disease, and atherosclerosis.

In the present invention, "fatty liver" refers to a condition or disease in which fat accumulates in excessive amounts in liver cells due to a disorder of fat metabolism in the liver.

In the present invention, "hyperlipidemia" means a condition or disease in which the concentration of fatty components in the blood, in particular cholesterol and triglycerides, is high compared to normal levels, and is used in a broad sense to include any condition requiring a reduction in the concentration of lipids in the blood.

In the present invention, "arteriosclerosis" means a condition or disease in which blood circulation to the organs and tissues of the body is impaired due to thickening and decreased elasticity of the arterial walls, and includes the term "atherosclerosis," which means a condition or disease in which blood circulation is impaired due to the deposition of fat, cholesterol, and other substances on the lining of the arteries, forming plaques, which narrow the lumen. Atherosclerosis can occur anywhere in the body, and when it occurs in the blood vessels of the heart, it can lead to coronary artery disease such as angina and myocardial infarction; when it occurs in the brain, it can lead to cerebral infarction; and when it occurs in the kidneys, it can lead to kidney failure.

Meanwhile, the strength, binding, and expression of Foxp3, T cell receptor (TCR) on Tregs, their ability to produce anti-inflammatory cytokines such as interleukin-10 (IL-10) and IL-35, cytotoxic T-lymphocyte-associated protein 4 (CTLA-4), programmed cell death protein 1 (PD-1), CD39, and CD73 are known to play an important role in immune regulation.

Recent studies have shown that the expression levels of these proteins are related to metabolism. That is, after Treg activation, mTOR signaling is upregulated, leading to lipid synthesis, mevalonate metabolism, and mitochondrial function, while CDTLA-4 and PD-1 have both been described to block PD-1 signaling, which promotes lipolysis and fatty acid oxidation, CD39, which converts adenosine triphosphate (ATP) and adenosine diphosphate (ADP) to adenosine monophosphate (AMP), and CD73, which converts AMP to adenosine, eliminate ATP effects such as P2 receptor-mediated cytotoxicity and ATP-induced maturation of dendritic cells.

Alterations in Treg numbers and function have been widely demonstrated in human autoimmune, infectious, and allergic diseases and cancer. Furthermore, reduced Treg levels have been reported in patients with type 2 diabetes and contribute to both hyperglycemia and high-density lipoprotein concentrations in the blood. Circulating adipose and visceral Tregs are reduced in obese individuals and inversely correlate with measures of adiposity, inflammation, and glucose tolerance, allowing identification of subjects at increased metabolic and cardiovascular risk. PPARγ signaling of Treg to energy homeostasis maintains the inflammatory state of adipose tissue and insulin sensitivity of lean adipose tissue, while dysfunction attenuates insulin sensitization. Interestingly, although attenuation of acute graft-versus-host disease and multiple sclerosis was observed in animal models, the frequency and suppressive capacity of Treg remained unchanged after silencing of INSR.

Thus, alterations in the number and function of Tregs are closely related to autoimmune and cancer diseases, as well as metabolic diseases including type 2 diabetes, obesity, and cardiovascular disease. And binding molecules that specifically bind to the Lrig-1 protein or epitopes thereof present on Tregs, which can alter the number or function of Tregs, may prevent or treat metabolic diseases including type 2 diabetes, obesity, and cardiovascular disease.

Still another embodiment of the present invention is to provide pharmaceutical compositions for the prevention or treatment of various diseases, for example metabolic diseases, comprising an antibody-drug conjugate as an active ingredient.

In the present invention, the antibody or antigen-binding fragment or antibody-drug conjugates according to the present invention is capable of binding specifically to an epitope comprising a polypeptide represented by any one of the amino acid sequences of SEQ ID NOs: 35 to 45, which is present on regulatory T cells, thereby regulating the function of the regulatory T cells and regulating the activity of effector T cells, thus preventing or treating various diseases, such as metabolic diseases.

In the present invention, the "antibody-drug conjugate (ADC)" refers to a form in which the drug and the antibody are chemically linked to each other without degrading biological activity of the antibody and the drug. In the present invention, the antibody-drug conjugate denotes a form in which the drug is bound to an amino acid residue at the N-terminus of the heavy and/or light chain of the antibody, specifically, a form in which the drug is bound to an α-amine group at the N-terminus of the heavy and/or light chain of the antibody.

In the present invention, the "drug" may mean any substance having a certain biological activity for a cell, which is a concept including DNA, RNA, or a peptide. The drug may be in a form which contains a reactive group capable of reacting and crosslinking with an α-amine group, and also includes a form which contains a reactive group capable of reacting and crosslinking with an α-amine group and to which a linker is linked.

In the present invention, examples of the reactive group capable of reacting and crosslinking with the α-amine group are not limited to particular types, as long as the reactive group can react and crosslink with an α-amine group at the N-terminus of a heavy or light chain of an antibody. Examples of the reactive group include all types of groups known in the art, which are capable of reacting with an amine group. The reactive group may, for example, be any one of isothiocyanate, isocyanate, acyl azide, NHS ester, sulfonyl chloride, aldehyde, glyoxal, epoxide, oxirane, carbonate, aryl halide, imidoester, carbodiimide, anhydride, and fluorophenyl ester, without being limited thereto.

In the present invention, the antibody-drug conjugate comprises the antibody or antigen-binding fragment that binds specifically to the epitope of the present invention, that is, Lrig-1 protein, or an epitope comprising a polypeptide consisting of a partial amino acid sequence of the extracellular domain of the Lrig-1 protein, or an epitope comprising a polypeptide represented by any one of the amino acid sequences of SEQ ID NOs: 7 to 17. Here, the drug may include any drug that can treat a disease targeted by the antibody that binds specifically to the Lrig-1 protein. For example, the drug may be, but is not limited to, a drug that can treat metabolic diseases.

In the present invention, "metabolic disease" refers to a condition or disease that is closely related to, or caused by, obesity, and may be one or more selected from the group consisting of fatty liver, type 2 diabetes, hyperlipidemia, cardiovascular disease, and atherosclerosis.

Another embodiment of the present invention provides a pharmaceutical composition for preventing or treating metabolic disease comprising, as an active ingredient, a chimeric antigen receptor (CAR) comprising an antigen-specific binding domain, a linking domain, and a CD3 zeta (ζ) signaling domain.

In the present invention, the term "chimeric antigen receptor" or "CAR" refers to an engineered receptor comprising an extracellular antigen-binding domain an intracellular signaling domain. Although the most common type of CAR comprises a single chain variable fragment (scFv) derived from a monoclonal antibody fused to a transmembrane and intracellular domain of a T cell co-receptor such as the CD3 zeta (ζ) chain, the invention described herein is not limited to these domains. Rather, as used herein, "chimeric antigen receptor" or "CAR" refers to any receptor engineered to express any intracellular signaling molecule and an extracellular antigen-binding domain fused or linked to any intracellular signaling molecule. In the present invention, the binding domain may comprise a single-chain variable fragment (scFv) capable of specifically recognizing Lrig-1 protein. In the present invention, the "single-chain variable fragment" or "scFv" refers to a fusion protein of the variable heavy (VH) and variable light (VL) chains of an antibody with a peptide linker between the VL and VH.

In addition, in the present invention, the VH domain and the VL domain may be linked together by a flexible linker. In the present invention, the flexible linker may be a glycine/serine linker of about 10 to 30 amino acids (e.g., 30, 25, 20, 19, 18, 17, 16, 15, 14, 13, 12, 11, 10, 9, 8, 7, 6 or 5 amino acids), preferably 15 amino acids in length. In the present invention, the length of the linker may be an important determinant of the chimeric antigen receptor. Thus, linkers having a shorter length than the lower limit of the above range can enhance affinity but can also lead to intracellular multimer formation, thus impairing expression of the CAR. On the other hand, linkers having a longer length than the upper limit of the above range may decrease antigen affinity by moving the VL and VH CDRs further apart in space.

The chimeric antigen receptor of the present invention may further comprise at least one of a hinge region (or spacer) and a signaling domain. In the present invention, the hinge region is a portion connecting the antigen binding domain and the transmembrane domain, and is also called spacer. The hinge region has the purpose for expanding the antigen binding domain from T cell membrane or NK cell membrane. In the present invention, the hinge region may be obtained, for example, from any suitable sequence from any genus, including human or a part thereof, or may include a hinge region of a human protein including CD8, CD28, 4-1BB, OX40, all or part of CD3 zeta (ζ) chain, T cell receptor α or β chain, CD28, CD3ε, CD45, CD4, CD5, CD8, CD9, CD16, CD22, CD33, CD37, CD64, CD80, CD86, CD134, CD137, ICOS, CD154, functional derivatives thereof, or combinations thereof, which are commonly used in the art, without being limited thereto. In addition, in the present invention, the hinge region may comprise one selected from, but not limited to, immunoglobulin (e.g., IgG1, IgG2, IgG3, IgG4, and IgD).

In the present invention, the signaling domain refers to the portion of the chimeric antigen receptor that is found or is engineered to be found inside a T cell. In the present invention, the signaling domain may or may not also contain a transmembrane domain which functions to anchor the chimeric antigen receptor in the plasma membrane of a T cell. In the present invention, the transmembrane domain and the signaling domain may be derived from the same protein (e.g., CD3ζ molecule). Alternatively, the transmembrane domain and the signaling domain may be derived from different proteins (e.g., the transmembrane domain of a CD28 and the intracellular signaling domain of a CD3ζ molecule, or vice versa).

In the present invention, the transmembrane domain may comprise, for example, a T cell receptor α or β chain, all or part of CD3 zeta (ζ) chain, CD28, CD3ε, CD45, CD4, CD5, CD8, CD9, CD16, CD22, CD33, CD37, CD64, CD80, CD86, CD134, CD137, ICOS, CD154, functional derivatives thereof, or combinations thereof, without being limited thereto. In the present invention, the costimulatory domain may comprise a functional signaling domain derived from a polypeptide comprising a ligand that binds to 4-1BB (CD137), OX40, CD27, CD28, CD30, CD40, PD-1, CD2, CD7, CD258, natural killing group 2 member C (NKG2C), natural killing group 2 member D (NKG2D), B7-H3, CD83, ICAM-1, LFA-1 (CD11a/CD18), or ICOS, active fragments thereof, functional derivatives thereof, or combinations thereof, without being limited thereto.

In the present invention, the signaling domain may comprise a functional signaling domain derived from a polypeptide comprising all or part of CD3 zeta (ζ), common FcR gamma (FcERIG), Fc gamma RIIIa, FcR beta (Fc epsilon R1b), CD3 gamma, CD3 delta, CD3 epsilon, CD79a, CD79b, DNAX-activating protein 10 (DAP10), DNAX-activating protein 12 (DAP12), active fragments thereof, functional derivatives thereof, or combinations thereof, without being limited thereto. Such signaling domains are known in the art.

In the present invention, "metabolic disease" refers to a condition or disease that is closely related to, or caused by, obesity, and may be one or more selected from the group consisting of fatty liver, type 2 diabetes, hyperlipidemia, cardiovascular disease, and atherosclerosis.

Meanwhile, in the prevention, "prevention" may include, without limitation, any act of blocking symptoms of a disease, or suppressing or delaying the symptoms, using the pharmaceutical composition of the present invention.

In addition, in the present invention "treatment" may include, without limitation, any act of ameliorating or beneficially altering symptoms of a disease, using the pharmaceutical composition of the present invention.

In the present invention, the pharmaceutical composition may be characterized by being in the form of capsules, tablets, granules, injections, ointments, powders, or beverages, and the pharmaceutical composition may be characterized by being intended for human subjects.

In the present invention, for use, the pharmaceutical composition may be formulated in the form of oral preparations such as powders, granules, capsules, tablets, and aqueous suspensions, preparations for external use, suppositories, and sterile injectable solutions, according to respective conventional methods, without being limited thereto. The pharmaceutical composition of the present invention may further comprise a pharmaceutically acceptable carrier. As the pharmaceutically acceptable carrier, a binder, a glidant, a disintegrant, an excipient, a solubilizer, a dispersant, a stabilizer, a suspending agent, a pigment, a flavor, and the like may be used for oral administration; a buffer, a preserving agent, a pain-relieving agent, a solubilizer, an isotonic agent, a stabilizer, and the like may be used in admixture for injections; and a base, an excipient, a lubricant, a preserving agent, and the like may be used for topical administration. The pharmaceutical composition of the present invention may be prepared in various dosage forms by being mixed with the pharmaceutically acceptable carrier as described above. For example, for oral administration, the pharmaceutical composition may be formulated in the form of tablets, troches, capsules, elixirs, suspensions, syrups, wafers, or the like. For injections, the pharmaceutical composition may be formulated in the form of unit dosage ampoules or multiple dosage forms. Alternatively, the pharmaceutical composition may be formulated into solutions, suspensions, tablets, capsules, sustained-release preparations, or the like.

Meanwhile, examples of carriers, diluents, or excipients suitable for making preparations include lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, mineral oil, or the like. In addition, a filler, an anti-coagulant, a lubricant, a wetting agent, a fragrance, an emulsifier, a preservative, and the like may further be included.

The route of administration of the pharmaceutical composition of the present invention includes, but is not limited to, oral, intravenous, intramuscular, intraarterial, intramedullary, intradural, intracardiac, transdermal, subcutaneous, intraperitoneal, intranasal, intestinal, topical, sublingual, or rectal route. Oral or parenteral administration is preferred.

In the present invention, the "parenteral" includes subcutaneous, intradermal, intravenous, intramuscular, intraarticular, intrabursal, intrasternal, intradural, intralesional, and intracranial injection or infusion techniques. The pharmaceutical composition of the present invention may also be administered in the form of suppositories for rectal administration.

The pharmaceutical composition of the present invention may vary depending on a variety of factors, including the activity of a certain compound used, the patient's age, body weight, general health status, sex, diet, the time of administration, the route of administration, the rate of excretion, drug combination, and the severity of a certain disease to be prevented or treated. Although the dose of the pharmaceutical composition may vary depending on the patient's condition and body weight, the severity of disease, the drug form, the route of administration, and the duration of administration, it may be appropriately selected by those skilled in the art. The pharmaceutical composition may be administered at a dose of 0.0001 to 50 mg/kg/day or 0.001 to 50 mg/kg/ day. Administration may be made once a day or several times a day. The dose is not intended to limit the scope of the invention in any way. The pharmaceutical composition according to the present invention may be formulated in the form of pills, sugar-coated tablets, capsules, liquids, gels, syrups, slurries, or suspensions.

Another embodiment of the present invention is directed to a method for preventing or treating metabolic disease, the method comprising a step of administering, to a subject, a pharmaceutically effective amount of the binding molecule according to the present invention, or the antibody-drug conjugate (ADC), or the chimeric antigen receptor (CAR).

In the present invention, "metabolic disease" refers to a condition or disease that is closely related to, or caused by, obesity, and may be one or more selected from the group consisting of fatty liver, type 2 diabetes, hyperlipidemia, cardiovascular disease, and atherosclerosis.

The antibody or antigen-binding fragment according to the present invention and the antibody-drug conjugate according to the present invention are capable of binding specifically to an epitope comprising a polypeptide represented by any one of the amino acid sequences of SEQ ID NOs: 35 to 45, thereby controlling the function of regulatory T cells and regulating the activity of effector T cells, thus very effectively preventing or treating various diseases.

In the present invention, the "subject" is a subject suspected of developing metabolic disease. The subject suspected of developing metabolic disease refers to mammals, such as humans, mice, and domestic animals, which had or are likely to develop the disease in question. However, the subject includes, without limitation, any subject treatable with the antibody, antigen-binding fragment or antibody-drug conjugate of the present invention.

The method of the present invention may comprise administering a pharmaceutically effective amount of the antibody or the antibody-drug conjugate. An appropriate total daily amount used may be determined by an attending physician within the scope of sound medical judgment, and administration may be made once or several times. However, for the purposes of the present invention, a specific therapeutically effective amount for a particular patient is preferably applied differently depending on various factors, including the type and degree of reaction to be achieved, the specific composition including whether other agents are used therewith as the case may be, the patient's age, body weight, general health status, sex, and diet, the time of administration, the route of administration, the secretion rate of the composition, the duration of treatment, and drugs used simultaneously or in combination with the specific composition, and similar factors well known in the medical field.

Meanwhile, the method for preventing or treating disease may be, but is not limited to, a combination therapy that further comprises administering a compound or substance having therapeutic activity against one or more diseases.

In the present invention, the "combination" should be understood to represent simultaneous, individual, or sequential administration. In a case where the administration is made in a sequential or individual manner, the second component should be administered at intervals such that beneficial effects of the combination are not lost.

In the present invention, the dose of the antibody or antibody-drug conjugate may be, but is not limited to, about 0.0001 µg to 500 mg per kg of patient's body weight.

According to another embodiment of the present invention, there is provided a diagnostic composition for a metabolic disease comprising an agent for measuring the expression level of the Lrig-1 protein or its extracellular domain or a gene encoding the Lrig-1 protein present on the surface of a T cell using the binding molecule of the present invention.

The T cells of the present invention may be regulatory T cells.

The diagnostic composition of the present invention may comprise, for example, an agent that measures the expression level of the Lrig-1 protein or its extracellular domain or a gene encoding the Lrig-1 protein present on the surface of an activated regulatory T cell, that is, regulatory T cells with activated suppression of effector T cells, but is not limited thereto.

The diagnostic composition of the present invention may further comprise a protein, more than one selected from the group consisting of CD25, TIGIT, LAG3, CTLA-4, GITR, OX40, ICOS, PD-1, TIM-3, CCR4, FR4, CD15s, PI-16, and Lrig-1 proteins present on the surface of T cells, and an agent for measuring the expression level of a gene encoding the same. As such, when the expression level of various proteins or genes encoding such is further measured on the surface of T cells, a significant synergistic effect can be achieved in diagnosing the target disease compared to measuring the Lrig-1 protein or its extracellular domain or gene encoding it alone.

Although the agent measuring the expression level of the Lrig-1 protein or its extracellular domains of the present invention, and the expression level of more than one protein selected from the group consisting of CD25, TIGIT, LAG3, CTLA-4, GITR, OX40, ICOS, PD-1, TIM-3, CCR4, FR4, CD15s, PI-16, and Lrig-1 protein are not particularly limited, it may comprise, for example, at least one selected from the group consisting of an antibody, oligopeptide, ligand, peptide nucleic acid (PNA), and aptamer that specifically binds to the protein.

As used herein, "peptide nucleic acid (PNA)" refers to an artificially synthesized, DNA- or RNA-like polymer, first introduced in 1991 by Professors Nielsen, Egholm, Berg and Buchardt at the University of Copenhagen, Denmark. Whereas DNA has a phosphate-ribose sugar backbone, PNA has a repeated N-(2-aminoethyl)-glycine backbone linked by peptide bonds, which greatly increases its binding to DNA or RNA and stability, leading to its use in molecular biology, diagnostic assays, and antisense therapeutics. PNAs have been described in the literature [Nielsen PE, Egholm M, Berg RH, Buchardt O (December 1991). "Sequence-selective recognition of DNA by strand displacement with a thymine-substituted polyamide". Science 254 (5037): 1497-1500], which is disclosed in detail herein.

As used herein, an "aptamer" is an oligonucleotide or peptide molecule, and a general description of aptamers can be found in the literature [Bock LC et al, Nature 355(6360):5646 (1992); Hoppe-Seyler F, Butz K "Peptide aptamers: powerful new tools for molecular medicine". J Mol Med. 78(8):42630(2000); Cohen BA, Colas P, Brent R. "An artificial cell-cycle inhibitor isolated from a combinatorial library". Proc Natl Acad Sci USA. 95(24): 142727 (1998)].

The agent measuring the expression level of the gene encoding the Lrig-1 protein of the present invention (in other words, LAIR1) or the gene encoding the extracellular domain of the Lrig-1 protein, and a gene encoding more than one protein selected from the group consisting of CD25, TIGIT, LAG3, CTLA-4, GITR, OX40, ICOS, PD-1, TIM-3, CCR4, FR4, CD15s, PI-16, and Lrig-1 may comprise at least one selected from the group consisting of a primer, probe, LNA, and antisense nucleotides that specifically bind to the gene.

As used herein, the term "primer" refers to a fragment that recognizes a target gene sequence, and consists of a forward and reverse primer pair, but preferably provides analysis results with specificity and sensitivity. High specificity can be imparted when the primers amplify the target gene sequence containing complementary primary binding sites while not causing non-specific amplification because the nucleic acid sequence of the primer is inconsistent with the non-target sequence present in the sample.

As used herein, the term "probe" refers to a substance that can specifically bind to a target substance to be detected in a sample, and a substance that can specifically confirm the presence of a target substance in a sample through binding. Given probes are commonly used in the art, the type of probe is not limited, but preferably can be peptide nucleic acid (PNA), locked nucleic acid (LNA), peptide, polypeptide, protein, RNA, or DNA, with PNA being the most preferred. More specifically, the probe is a biomaterial that includes those derived from or similar to those from living organisms or those manufactured in vitro, for example, enzymes, proteins, antibodies, microorganisms, animal and plant cells and organs, nerve cells, DNA, and RNA. DNA may comprise cDNA, genomic DNA and oligonucleotides, while RNA may comprise genomic RNA, mRNA, oligonucleotides, while proteins may comprise antibodies, antigens, enzymes, peptides, and the like.

As used herein, "locked nucleic acids (LNA)" refers to nucleic acid analogs comprising a 2'-O, 4'-C methylene bridge [J Weiler, J Hunziker and J Hall Gene Therapy (2006) 13, 496.502]. LNA nucleosides include the common nucleic acid bases of DNA and RNA, and can form base pairs according to the Watson-Crick base pairing rule. However, due to the "locking" of the molecule due to the methylene bridge, LNA does not form the ideal conformation in Watson-Crick bonds. When LNA is incorporated into DNA or RNA oligonucleotides, LNA can pair more rapidly with complementary nucleotide chains to increase the stability of the double helix.

As used herein, "antisense" refers to an oligomer having a sequence of nucleotide bases and an inter-subunit backbone that allows the antisense oligomer to hybridize to a target sequence in RNA by Watson-Crick base-pairing, resulting in the formation of an mRNA:RNA:oligomer heterodimer typically within the target sequence. The oligomer may have exact sequence complementarity or approximate sequence complementarity to the target sequence.

Since the Lrig1 protein or the gene encoding it (LRIG1) according to the present invention is known, those skilled in the art will be able to use it to facilitate the design of primers, probes or antisense nucleotides that specifically bind to the gene encoding said protein.

According to another embodiment of the invention, the present invention relates to a kit for the diagnosis of a metabolic disease comprising the antibody or antigen-binding fragment provided in the present invention.

In the present invention, the kit may be, but is not limited to, an RT-PCR kit, a DNA chip kit, an ELISA kit, a protein chip kit, a rapid kit, or a multiple reaction monitoring (MRM) kit.

The kits of the present invention may further comprise one or more other component compositions, solutions, or devices suitable for the assay method.

The diagnostic kit of the present invention may further include one or more other component compositions, solutions, or devices suitable for assay methods. For example, the diagnostic kit of the present invention may further include essential elements required for performing reverse transcription polymerase reaction. The kit for reverse transcription polymerase reaction includes a pair of primers specific for a gene encoding a marker protein. The primer pair is a nucleotide having a sequence specific to a nucleic acid sequence of the gene, and may have a length of about 7 bp to 50 bp, or about 10 bp to 30 bp. The kit may also include a primer pair specific for a nucleic acid sequence of a control gene. In addition to those mentioned above, the kit for reverse transcription polymerase reaction may include test tubes or other suitable containers, reaction buffers (with varying pH and magnesium concentrations), deoxynucleotides (dNTPs), enzymes such as Taq-polymerase and reverse transcriptase, DNase and RNase inhibitors, DEPC-treated water, sterilized water, and the like.

In addition, the diagnostic kit of the present invention may include essential elements required for performing a DNA chip assay. The DNA chip kit may include a substrate to which a cDNA or oligonucleotide corresponding to a gene or a fragment thereof is attached; reagents, agents, and enzymes for preparing a fluorescence-labeled probe; and the like. The substrate may also contain a cDNA or oligonucleotide corresponding to a control gene or a fragment thereof.

In addition, the diagnostic kit of the present invention may include essential elements required for performing ELISA. The ELISA kit includes an antibody specific for the marker protein. The antibodies are antibodies with high specificity and affinity for the marker protein and little cross-reactivity with other proteins, and include monoclonal antibodies, polyclonal antibodies, or recombinant antibodies. The ELISA kit may also include an antibody specific for a control protein. In addition to those mentioned above, the ELISA kit may include reagents capable of detecting bound antibodies, for example, labeled secondary antibodies, chromophores, enzymes (which are, for example, conjugated with an antibody) and substrates thereof, and other substances capable of binding to antibodies.

According to another embodiment of the present invention, the present invention relates to a method for providing information on diagnosis of an metabolic disease comprising a step of measuring the expression level of Lrig-1 protein or extracellular domain thereof present on the surface of T cells in a biological sample isolated from a target subject using the binding molecule of the present invention, or the expression level of a gene encoding such.

The T cells of the present invention may be regulatory T cells.

The step of measuring the expression level of the present invention may be to further measure the expression levels of a protein, more than one selected from the group consisting of CD25, TIGIT, LAG3, CTLA-4, GITR, OX40, ICOS, PD-1, TIM-3, CCR4, FR4, CD15s, PI-16, and Lrig-1 proteins present on the surface of T cells, or the expression levels of a gene encoding the same. As such, when the expression level of various proteins or genes encoding such is further measured on the surface of T cells, a significant synergistic effect can be achieved in diagnosing the target disease compared to measuring the Lrig-1 protein or the gene encoding it alone.

In the present invention, the "target individual" refers to an individual for whom it is uncertain whether the disease has developed and who has a high probability of developing the disease.

In the present invention, the "biological sample" may refer to any material, biological fluid, tissue or cell obtained or derived from an individual, and can comprise, for example, whole blood, leukocytes, peripheral blood mononuclear cells, buffy coat, plasma, serum, sputum, tears, mucus, nasal washes, nasal aspirates, breath, urine, semen, saliva, peritoneal washings, pelvic fluids, cystic fluid, meningeal fluid, amniotic fluid, glandular fluid, pancreatic fluid, lymph fluid, pleural fluid, nipple aspirate, bronchial aspirate, synovial fluid, joint aspirate, organ secretions, cells, cell extracts, or cerebrospinal fluid, but is not limited thereto.

The Lrig-1 protein or extracellular domain thereof of the present invention may be expressed on the cell surface of T cells, in particular, regulatory T cells, for example, activated regulatory T cells, that is the cell surface of activated regulatory T cells with activated suppressive ability of effector T cells.

In the present invention, examples of the method for measuring or comparatively analyzing the expression level of the Lrig-1 protein, extracellular domain thereof, and more than one protein selected from the group consisting of CD25, TIGIT, LAG3, CTLA-4, GITR, OX40, ICOS, PD-1, TIM-3, CCR4, FR4, CD15s, PI-16 and Lrig-1 include, but are not limited to, protein chip assay, immunoassay, ligand binding assay, matrix assisted laser desorption/ionization time of flight mass spectrometry (MALDI-TOF), sulface enhanced laser desorption/ionization time of flight mass spectrometry (SELDI-TOF), radioimmunoassay, radial immunodiffusion, ouchterlony immunodiffusion, rocket immunoelectrophoresis, immunohistochemical staining, complement fixation assay, two-dimensional electrophoresis, liquid chromatography-mass spectrometry (LC-MS), liquid chromatography-mass spectrometry/mass spectrometry (LC-MS/MS), Western blotting, and enzyme linked immunosorbent assay (ELISA).

The analysis method for measuring the expression level of a gene in order to confirm the presence and expression level of genes encoding the Lrig-1 protein or its extracellular domain, and more than one protein selected from the group consisting of CD25, TIGIT, LAG3, CTLA-4, GITR, OX40, ICOS, PD-1, TIM-3, CCR4, FR4, CD15s, PI-16 and Lrig-1 may be, but is not limited to, reverse transcription polymerase reaction (RT-PCR), competitive RT-PCR, real-time RT-PCR, RNase protection assay (RPA), Northern blotting or DNA chip.

In the present invention, the method may comprise a step of predicting that the target individual has a high probability of developing an metabloic disease in a case where the expression level of Lrig-1 protein or its extracellular domain, or the gene encoding it measured in the biological sample of the target subject is decreased compared to the normal control.

In the present invention, said "control" may be a normal control, or more specifically, may be obtained from a serum sample of a patient identified as having no metabolic disease, or may be an average to median value of the expression level of TregL1 in a patient who has undergone bone density testing, ultrasound, and CT and who is ultimately identified as having no metabolic disease. The expression level of the marker protein or gene encoding it in a control group can be compared to the expression level of the marker protein or gene encoding it in a biological sample derived from a patient developing the metabolic disease being analyzed, and a diagnosis of the metabolic disease can be made by determining whether there is a significant change in said expression level.

As used in the present invention, "high level" means measurably and significantly elevated expression relative to a normal control, and more specifically may mean, but is not limited to, e.g., 20% or more elevated expression relative to a normal control, more specifically may mean 30%, more specifically may mean 40%, and most specifically may mean 50% or more elevated expression relative to a normal control.

In the present invention, the method may comprise a step of predicting that the target individual has a high probability of developing an metabolic disease in a case where the expression level of more than one protein selected from the group consisting of CD25, TIGIT, LAG3, CTLA-4, GITR, OX40, ICOS, PD-1, TIM-3, CCR4, FR4, CD15s, PI-16 and Lrig-1 proteins, or where the expression level of the genes encoding such proteins is changed (increased or decreased) compared to the normal control.

In the present invention, the method may comprise a step of predicting that the target individual has a high probability of developing an metabolic disease in a case where the expression level of Lrig-1 protein or the gene encoding it is decreased compared to a normal control.

Furthermore, in the case of predicting or diagnosing that an metabolic disease is highly likely to occur by measuring the expression level of Lrig-1 protein or its extracellular domain or the gene encoding it, the method may further comprise a step of administering a drug for the disease to the individual.

As used in the present invention, "diagnosing" means determining the presence or characterization of a pathological condition, and more specifically, determining a metabolic disease.

Another embodiment of the invention relates to a diagnostic device for diagnosing a metabolic disease.

The measurement part of said diagnostic device of the present invention can measure the expression level of TregL1 using a preparation for measuring the expression level of TregL1 in a biological sample obtained from an objective individual.

In the present invention, the intended individual may be selected from the group consisting of humans, rats, mice, marmots, hamsters, rabbits, monkeys, dogs, cats, cattle, horses, pigs, sheep and goats, and may be specifically human, but is not limited thereto.

In the present invention, the "biological sample" may refer to any material, biological fluid, tissue or cell obtained or derived from an individual, and can comprise, for example, whole blood, leukocytes, peripheral blood mononuclear cells, buffy coat, plasma, serum, sputum, tears, mucus, nasal washes, nasal aspirates, breath, urine, semen, saliva, peritoneal washings, pelvic fluids, cystic fluid, meningeal fluid, amniotic fluid, glandular fluid, pancreatic fluid, lymph fluid, pleural fluid, nipple aspirate, bronchial aspirate, synovial fluid, joint aspirate, organ secretions, cells, cell extracts, or cerebrospinal fluid, but is not limited thereto.

The agent utilized in the measurement portion of the diagnostic device of the present invention may be an agent that measures the expression level of TregL1. More specifically, it may comprise one or more species selected from the group consisting of primers, probes and antisense nucleotides that specifically bind to said gene.

The presence or absence of a metabolic disease can be predicted by determining the degree of expression of said gene using said agent in the measurement portion of said diagnostic device of the present invention.

The diagnostic device for metabolic diseases of the present invention may further comprise a detection part that predicts and outputs the presence or absence of a metabolic disease of the subject from the degree of expression of said gene obtained in said measurement part.

In the present invention, said detection part may diagnose a metabolic disease by generating information and categorizing the subject as having developed or likely to develop a metabolic disease if the expression level of said gene obtained from said measurement part is higher than the expression level of TregL1 measured in a control group.

Furthermore, in the present invention, said detection part can diagnose a metabolic disease by generating information and categorizing a patient as having developed or likely to develop a metabolic disease if the expression level of said gene obtained from said measurement part is higher than the expression level of TregL1 measured in a control group.

According to one embodiment of the present invention, there is provided a composition for diagnosing a metabolic disease comprising an agent for measuring the expression level of a Lrig-1 protein.

According to one embodiment of the present invention, a diagnostic composition is provided wherein the Lrig-1 protein is present on the surface of an adipocyte.

According to one embodiment of the invention, there is provided a diagnostic composition, wherein the agent for measuring the expression level of said Lrig-1 protein comprises at least one species selected from the group consisting of a primer, a probe and an antisense nucleotide that specifically binds to said gene.

According to one embodiment of the present invention, an agent for measuring the expression level of said Lrig-1 protein provides a diagnostic composition, wherein said agent is an antibody or antigen-binding fragment that specifically binds to Lrig-1.

According to one embodiment of the present invention, providing a diagnostic composition, wherein said antibody or antigen-binding fragment is an antibody or antigen-binding fragment that specifically binds to at least one epitope selected from the group consisting of an amino acid sequence represented by SEQ ID NO: 35 to SEQ ID NO: 45.

According to one embodiment of the present invention, provides a diagnostic composition, wherein the antibody is a chimeric antibody, humanized antibody, bivalent, bispecific molecule, minibody, domain antibody, bispecific antibody, antibody mimetic, diabody, triabody, tetrabody, or fragment thereof.

According to one embodiment of the present invention, there is provided a diagnostic composition, wherein said metabolic disease is at least one selected from the group consisting of insulin resistance disease, obesity, diabetes, dyslipidemia, liver disease, kidney damage, arteriosclerosis, and hypertension.

According to one embodiment of the present invention, there is provided a kit for diagnosing a metabolic disease comprising the composition.

According to another embodiment of the present invention, there is provided a kit for diagnosing a metabolic disease, wherein said metabolic disease is at least one selected from the group consisting of insulin resistance disease, obesity, diabetes, dyslipidemia, liver disease, kidney damage, arteriosclerosis, and hypertension.

According to one embodiment of the present invention, there is provided a method of providing information for diagnosing a metabolic disease, comprising the step of measuring a level of Lrig-1 protein expression in a biological sample isolated from a subject.

According to another embodiment of the invention, a method of providing information, wherein the Lrig-1 protein is present on the surface of an adipocyte.

According to another embodiment of the present invention, a method of providing information, wherein the expression level of Lrig-1 protein measured in said biological sample is higher or lower than a normal control, predicts that the subject has a metabolic disease.

According to another embodiment of the present invention, a method of providing information wherein said metabolic disease is at least one selected from the group consisting of insulin resistance disease, obesity, diabetes, dyslipidemia, liver disease, kidney damage, arteriosclerosis, and hypertension.

According to another embodiment of the present invention, there is provided a diagnostic device for a metabolic disease comprising: a measurement part for measuring an expression level of Lrig-1 in a biological sample obtained from a subject; and a detection part for outputting, from the expression level of Lrig-1 measured in said measurement part, whether the subject has developed or is likely to develop a bone disease.

According to another embodiment of the present invention, there is provided a diagnostic instrument wherein said metabolic disease is at least one selected from the group consisting of insulin resistance disease, obesity, diabetes, dyslipidemia, liver disease, kidney damage, arteriosclerosis, and hypertension.

Another embodiment of the present invention provides a pharmaceutical composition for the prevention or treatment of a metabolic disease comprising as an active ingredient an antibody or antigen-binding fragment that specifically binds to Lrig-1.

According to another embodiment of the present invention, there is provided a pharmaceutical composition for the prevention or treatment of a metabolic disease comprising as an active ingredient a binding molecule that specifically binds to at least one epitope selected from the group consisting of an amino acid sequence represented by SEQ ID NO: 35 to SEQ ID NO: 45.

According to another embodiment of the present invention, there is provided a pharmaceutical composition wherein said binding molecule is an antibody or antigen-binding fragment.

According to another embodiment of the present invention, there is provided a pharmaceutical composition, wherein said antibody is a chimeric antibody, humanized antibody, bivalent, bispecific molecule, minibody, domain antibody, bispecific antibody, antibody mimetic, diabody, triabody, tetrabody, or fragment thereof.

According to another embodiment of the invention, a chimeric antigen receptor (CAR) comprising an antigen-specific binding domain, an association domain, and a CD3 zeta (ζ) signaling domain, wherein said antigen-specific binding domain specifically binds to at least one epitope selected from the group consisting of an amino acid sequence represented by SEQ ID NO: 35 to SEQ ID NO: 45; and a chimeric antigen receptor, which is a fusion protein comprising an immunoglobulin Fc region, as an active ingredient. Provided are pharmaceutical compositions for the prevention or treatment of metabolic diseases.

According to another embodiment of the present invention, there is provided a pharmaceutical composition for the prevention or treatment of a metabolic disease comprising as an active ingredient an antibody-drug conjugate comprising a binding molecule and a drug that specifically binds to at least one epitope selected from the group consisting of amino acid sequences represented by SEQ ID NO: 35 to SEQ ID NO: 45.

Another embodiment of the present invention provides a pharmaceutical composition, wherein the binding molecule is an antibody or antigen-binding fragment.

According to another embodiment of the present invention, there is provided a pharmaceutical composition, wherein said antibody is a chimeric antibody, humanized antibody, bivalent, bispecific molecule, minibody, domain antibody, bispecific antibody, antibody mimetic, diabody, triabody, tetrabody, or fragment thereof.

According to another embodiment of the present invention, there is provided a pharmaceutical composition, wherein said metabolic disease is at least one selected from the group consisting of insulin resistance disease, obesity, diabetes, dyslipidemia, liver disease, kidney damage, arteriosclerosis, and hypertension.

### Advantageous Effects

The antibody or antigen-binding fragment that binds specifically to the epitope of Lrig-1 protein according to the present invention is capable of binding specifically to the epitope of the present invention, which is present on regulatory T cells, thereby controlling the function of the regulatory T cells and controlling the activity of effector T cells. Thus, the antibody or the antigen-binding fragment may be very efficiently used to prevent, ameliorate or treat various diseases.

### Brief Description of Drawings

FIG. 1 shows the structure of Lrig-1 protein according to an embodiment of the present invention.
FIG. 2 shows the structure of Lrig-1 protein according to an embodiment of the present invention.
FIG. 3 shows the expression level of Lrig-1 mRNA according to an embodiment of the present invention.
FIG. 4 shows the expression level of Lrig-1 mRNA according to an embodiment of the present invention.
FIG. 5 shows the expression level of Lrig-1 mRNA according to an embodiment of the present invention.
FIG. 6 shows the expression level of Lrig-1, Lrig-2 and Lrig-3 mRNAs according to an embodiment of the present invention.
FIG. 7 shows the results of comparing expression levels of Lrig-1 protein in regulatory T cells and non-regulatory T cells according to an embodiment of the present invention.
FIG. 8 shows expression of Lrig-1 protein on the surface of regulatory T cells according to an embodiment of the present invention.
FIG. 9 shows the results of analyzing the binding capacities of antibodies (GTC210-01, GTC210-02, GTC210-03, GTC210-04, GTC110-01, GTC110-02, GTC110-03 and GTC110-04) to Lrig-1 protein in an embodiment of the present invention.
FIG. 10 shows the results of analyzing the mechanisms of regulating Lrig-1 protein-induced Stat3 phosphorylation in regulatory T cells by Lrig-1 protein-specific monoclonal antibodies (GTC210-01, GTC210-02, GTC210-03, GTC210-04, GTC110-01, GTC110-02, GTC110-03 and GTC110-04) in an embodiment of the present invention.

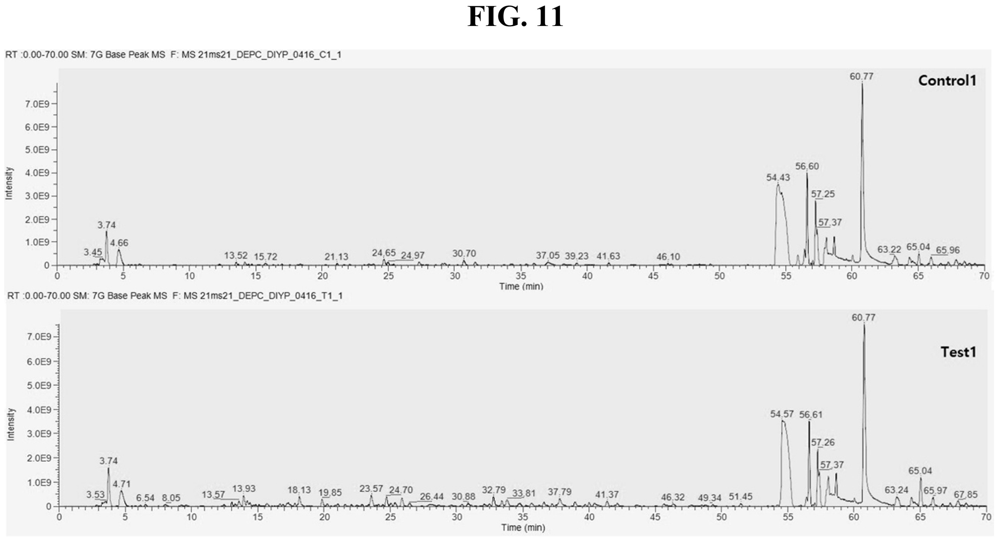

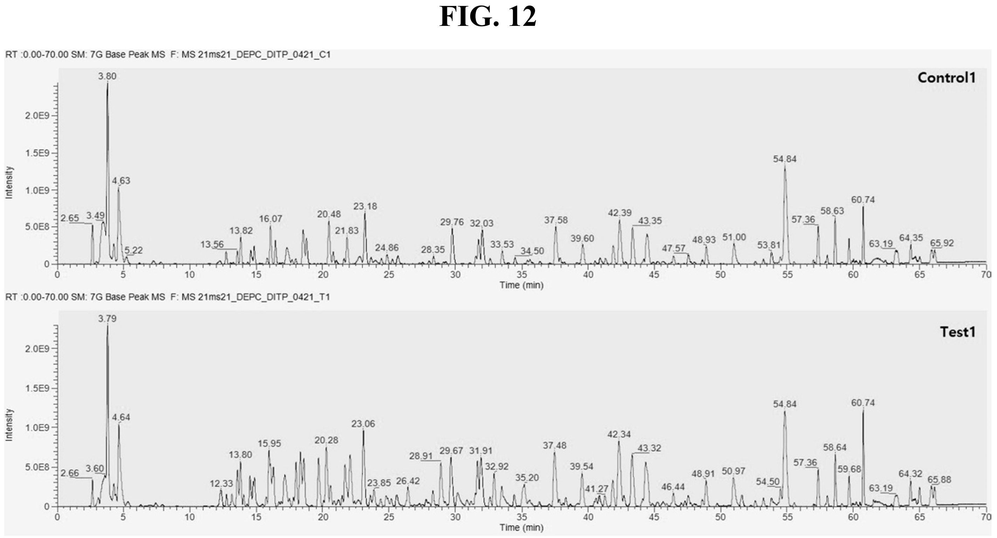

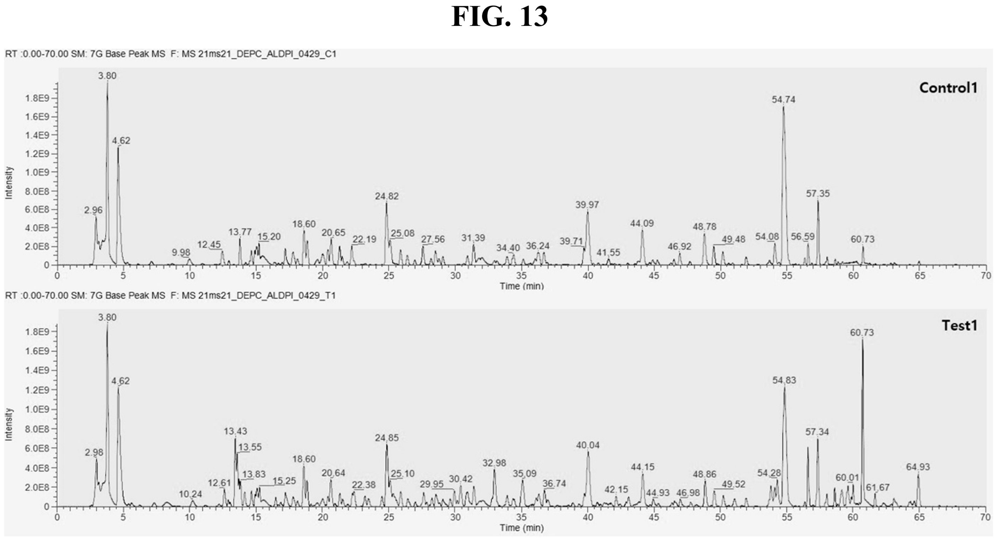

FIGS. 11 to 13 show the results of covalent labeling mass spectrometry of peptides degraded by treatment with proteases (chymotrypsin (FIG. 11), trypsin (FIG. 12), and Asp-N/Lys-C (FIG. 13)) according to an embodiment of the present invention.

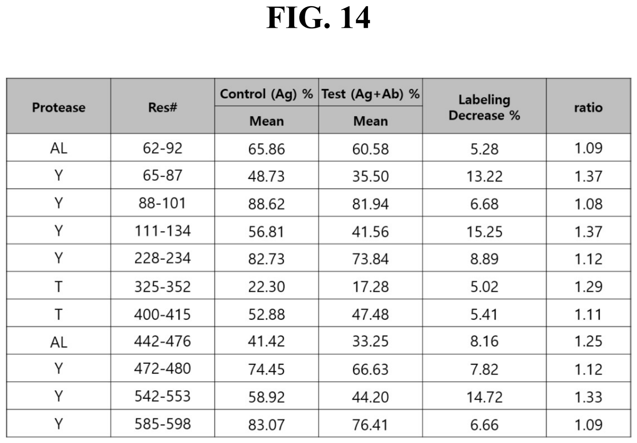

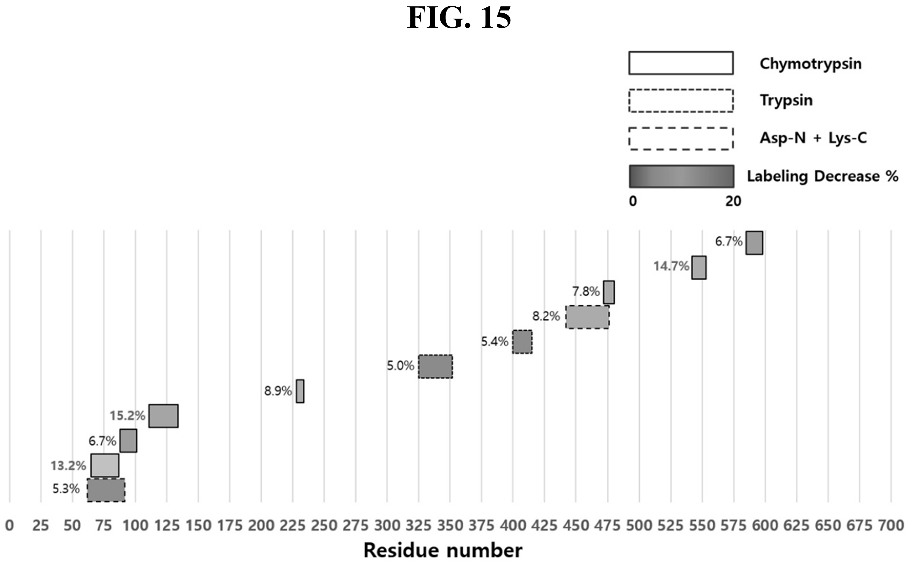

FIGS. 14 and 15 show the results of performing alignment and comparison based on the sequences identified by covalent labeling mass spectrometry of the peptides degraded by each protease according to an embodiment of the present invention.

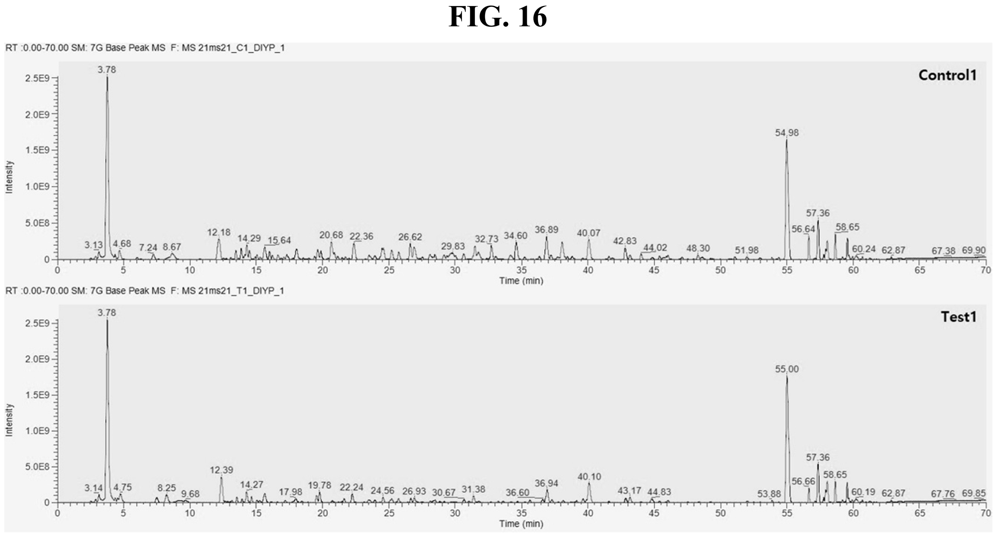

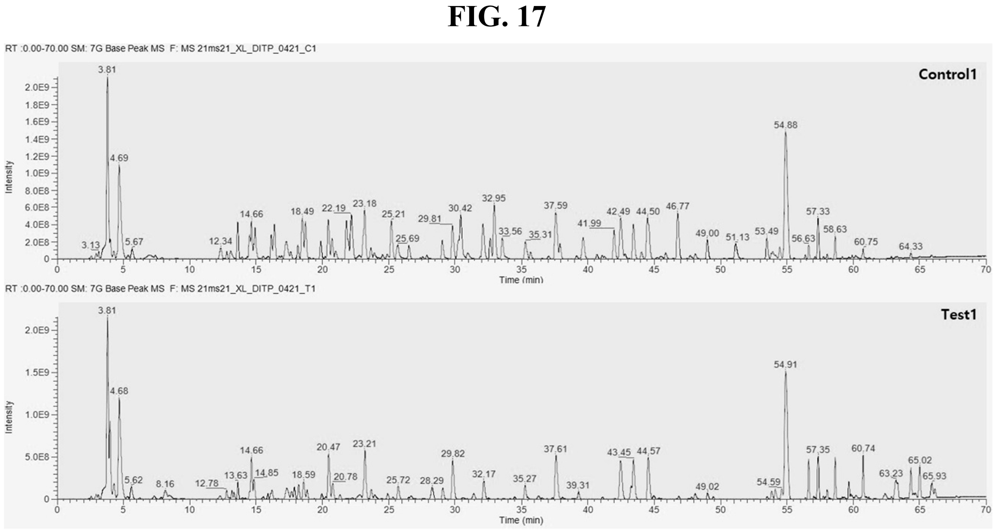

FIGS. 16 and 17 show the results of cross-linking assay of peptides degraded by treatment with proteases (chymotrypsin (FIG. 16) and trypsin (FIG. 17)) according to an embodiment of the present invention.

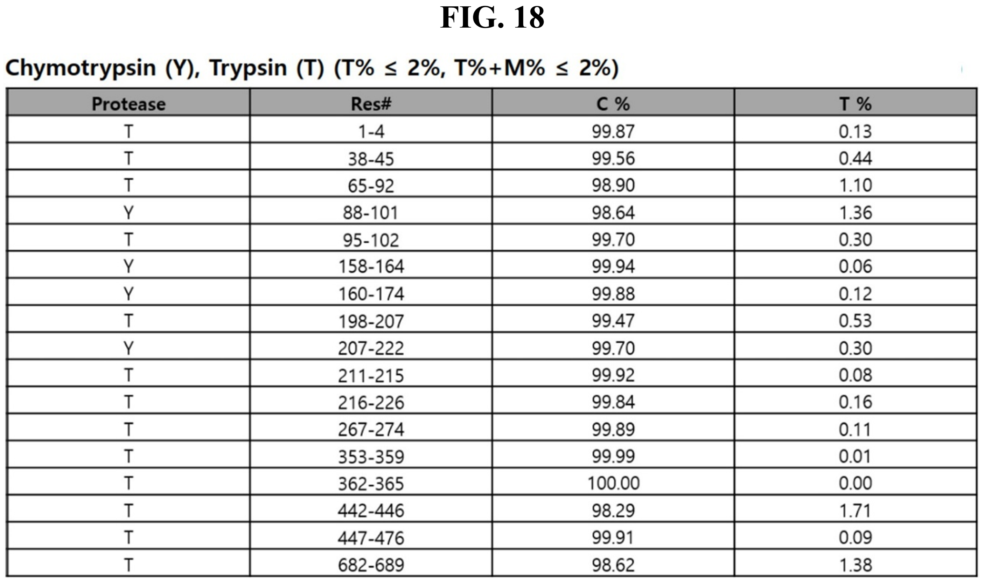

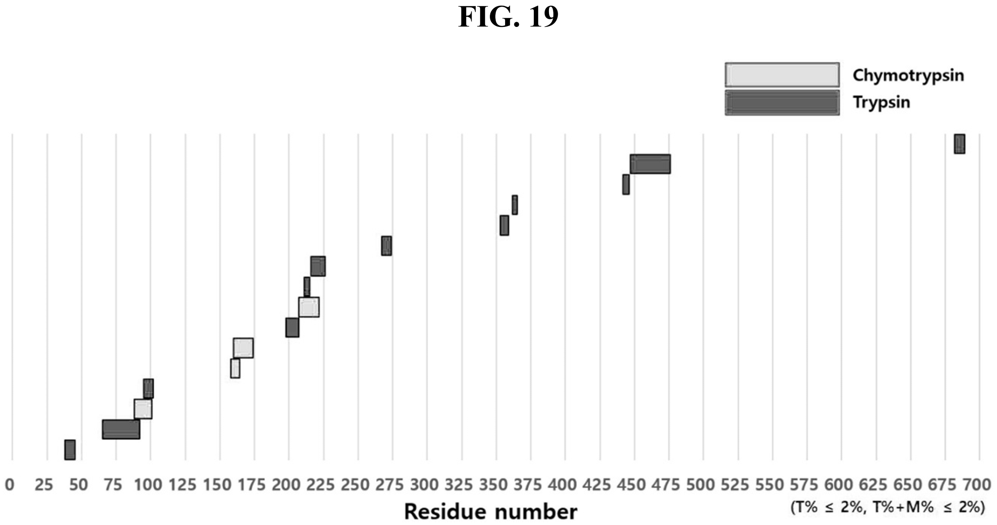

FIGS. 18 and 9 show the results of performing alignment and comparison based on the sequences identified by cross-linking assay of the peptides degraded by each protease according to an embodiment of the present invention.

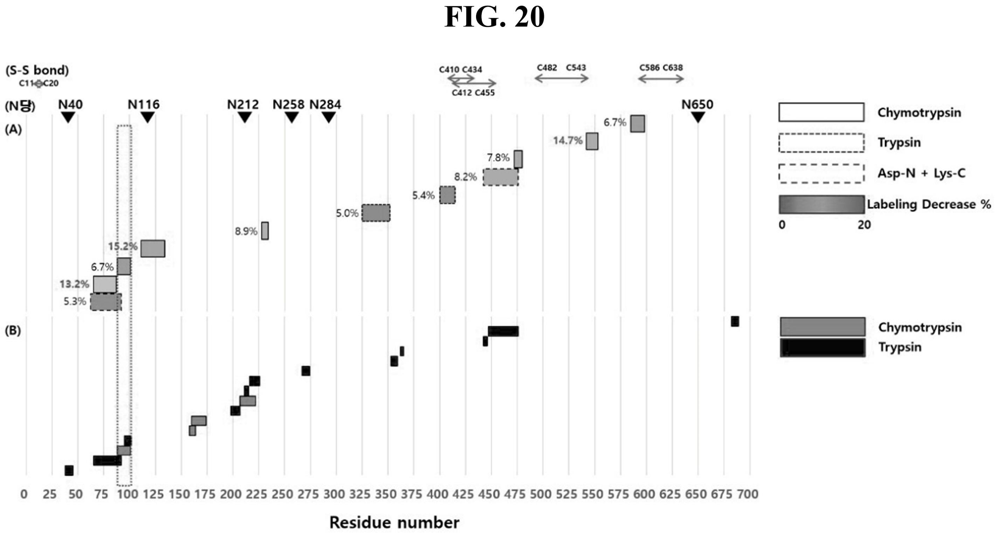

FIG. 20 shows the results of aligning and analyzing epitopes identified by covalent labeling mass spectrometry and cross-linking assay according to an embodiment of the present invention.
FIG. 21 shows the results of weight changes in Lrig-1 knockout mice for males and females.
FIG. 22 shows the weight change of the first identified Lrig-1 knockout male mice divided by whether the genotype is hetero or homo.
FIG. 23 shows the change in adipocytes in the first identified Lrig-1 knockout male mice, divided into visceral and subcutaneous fat. In the figure, PGF refers to eWAT, i.e. visceral fat, and SCF refers to iWAT, i.e. subcutaneous fat.
FIG. 24 shows the weight change of the first identified Lrig-1 knockout female mouse divided by whether the genotype is hetero or homo.
FIG. 25 shows the change in adipocytes in the first identified Lrig-1 knockout female mice, divided into visceral and subcutaneous fat. In this figure, PGF refers to eWAT, i.e. visceral fat, and SCF refers to iWAT, i.e. subcutaneous fat.
FIG. 26 shows the change in body weight of the second identified Lrig-1 knockout male mice, divided by genotype as hetero or homo.
FIG. 27 shows the change in adipocytes in the second Lrig-1 knockout male mouse, divided into visceral fat and subcutaneous fat. In this figure, PGF refers to eWAT, i.e. visceral fat, SCF refers to iWAT, i.e. subcutaneous fat, WAT refers to white adipose tissue, i.e. white adipocytes, and BAT refers to brown adipose tissue, i.e. brown adipocytes.
FIG. 28 shows the weight change of a second identified Lrig-1 knockout female mouse, divided by whether the genotype is hetero or homo.
FIG. 29 shows the change in adipocytes in the second identified Lrig-1 knockout female mouse, divided into visceral and subcutaneous fat. In the figure, PGF refers to eWAT, i.e. visceral fat, and SCF refers to iWAT, i.e. subcutaneous fat.
FIG. 30 illustrates the experimental process of differentiating a 3T3 cell line to express Lrig-1.
FIG. 31 shows the expression level of Lrig-1 as determined by flow cytometry.
FIG. 32 shows the expression level of Lrig-1 over time.

### Mode for Invention

Hereinafter, the present invention will be described in more detail with reference to examples. These examples are only for illustrating the present invention in more detail, and it will be obvious to those of ordinary skill in the art that the scope of the present invention according to the subject matter of the present invention is not limited by these examples.

### Examples

### [Preparation Example 1] T Cell Subset Culture

In order to identify whether the Lrig-1 protein is expressed only in regulatory T cells (Treg) cells, Th0, Th1, Th2, Th17 and iTreg, which are T cell subsets, were prepared. The iTreg refers to cells whose differentiation has been artificially induced in a medium having the following composition, unlike nTreg which has been naturally isolated.

The T cell subsets were obtained by isolating naive T cells from mouse spleens, and then inducing the isolated cells to differentiate into each T cell subset by 72 hours of culture in RPMI1640 (Invitrogen Gibco, Grand Island, NY) nutrient medium, which contained 10% fetal bovine serum (FBS; hyclone, logan, UT) and further contained the components shown in Table 1 below, in an incubator at 37°C under 5% CO₂.

**[Table 1]**

| Differentiated cells | Composition |
|---|---|
| Th0 | anti-CD3 and anti-CD28 |
| Th1 | IL-12 and anti-IL-4 antibodies |
| Th2 | IL-4 and anti-IFNβ |
| Th17 | IL-6, TGFβ, anti-IFNβ and anti-IL-4 |
| iTreg | IL-2 and TGFβ |

### [Example 11 Structural Analysis of Lrig-1

The three-dimensional steric structure of the extracellular domain of Lrig-1 protein was predicted to produce an antibody specific for the Lrig-1 protein, a protein present on the surfaces of regulatory T cells.

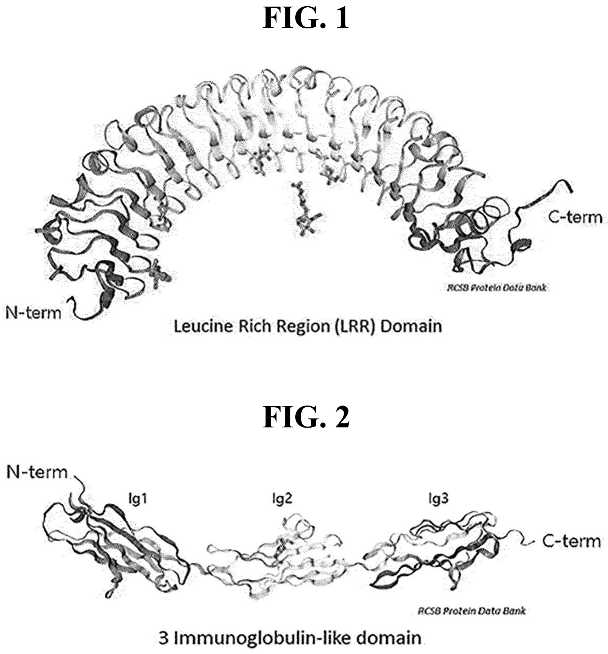

First, in order to predict the amino acid sequences of epitopes, tools of Uniprot (http://www.uniprot.org) and RCSB Protein Data Bank (http://www.rcsb.org/pdb) were used to predict the three-dimensional steric structure of the extracellular domain (ECD) of the Lrig-1 protein, and then the results are shown in FIGS. 1 and 2.

As shown in FIG. 1, a total of 15 leucine-rich regions (LRR1 to LRR15) were present in the Lrig-LRR domain (amino acid sequence at positions 41 to 494) in the extracellular domain of the Lrig-1 protein. Each of the LRR domains consisted of 23 to 27 amino acids, with 3 to 5 leucine residues being present.

In addition, as shown in FIG. 2, three immunoglobulin-like domains were present at positions 494 to 781 in the amino acid sequence of the Lrig-1 protein in the extracellular domain of the Lrig-1 protein.

### [Example 21 Identification of Specific Expression of Lrig-1 mRNA in Regulatory T Cells

It was verified whether the Lrig-1 protein could act as a biomarker specific for regulatory T cells.

For the verification, CD4⁺ T cells were isolated from mouse spleens by magnet-activated cell sorting (MACS) using CD4 beads. Thereafter, regulatory T (CD4⁺CD25⁺ T) cells and non-regulatory T (CD4⁺CD25⁻ T) cells were isolated by fluorescence activated cell sorting (FACS) using CD25 antibody. For the respective cells and the cells differentiated in Preparation Example 1, mRNA was extracted using Trizol, and then genomic DNA was removed from genomic RNA using a gDNA extraction kit (Qiagen) according to the protocol provided by the manufacturer. The mRNA from which gDNA has been removed was synthesized into cDNA using the BDsprint cDNA synthesis kit (Clonetech).

Real-time polymerase chain reaction (RT PCR) was performed to quantitatively analyze the expression level of Lrig-1 mRNA in the cDNA.

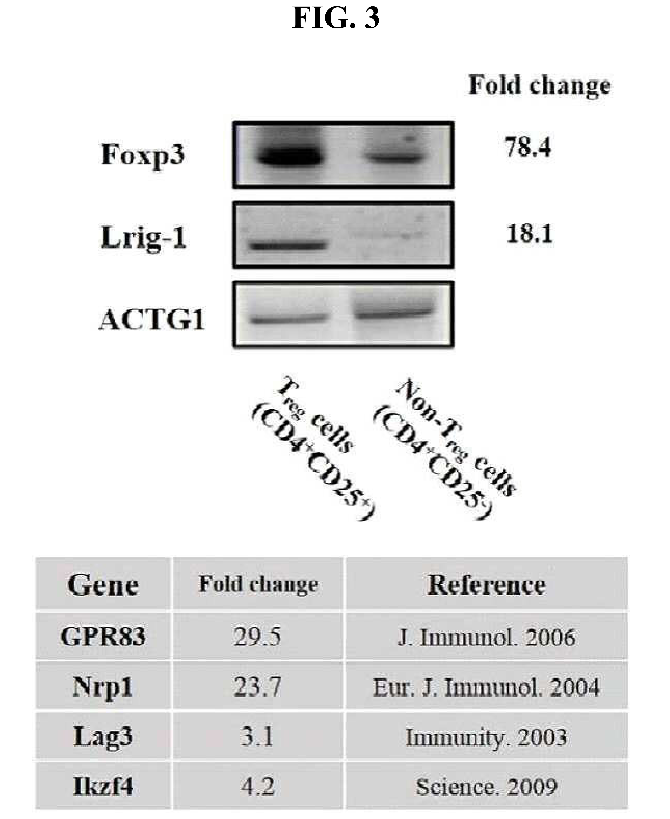

The real-time polymerase chain reaction was performed with primers shown in Table 2 below using SYBR Green (Molecular Probes) according to the protocol provided by the manufacturer for 40 cycles, each consisting of 95°C for 3 minutes, 61°C for 15 seconds, and 72°C for 30 seconds. The relative gene expression level was calculated using the ΔCT method and normalized using HPRT. The results are shown in FIGS. 3 to 6.

**[Table 2]**

| Primer | Direction | SEQ ID NO. |
|---|---|---|
| Mouse Lrig-1 | Forward | SEQ ID NO: 7 |
| | Reverse | SEQ ID NO: 8 |
| Mouse Lrig-2 | Forward | SEQ ID NO: 9 |
| | Reverse | SEQ ID NO: 10 |
| Mouse Lrig-3 | Forward | SEQ ID NO: 11 |
| | Reverse | SEQ ID NO: 12 |
| Mouse FOXP3 | Forward | SEQ ID NO: 13 |
| | Reverse | SEQ ID NO: 14 |
| ACTG1 | Forward | SEQ ID NO: 15 |
| | Reverse | SEQ ID NO: 16 |

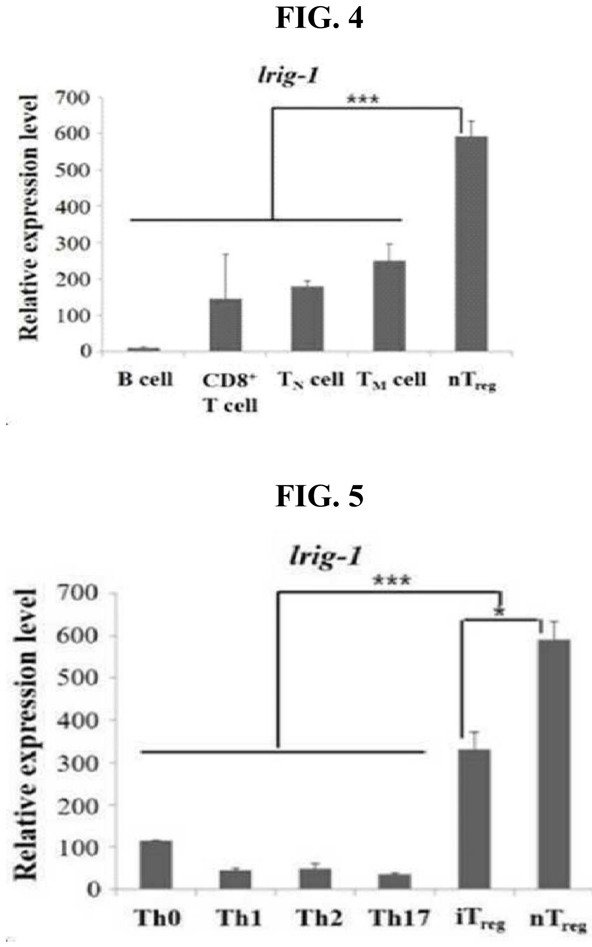

As shown in FIG. 3, it can be seen that the expression level of Lrig-1 in the regulatory T (CD4⁺CD25⁺ T) cells was 18.1 times higher than that in the non-regulatory T (CD4⁺CD25⁻ T) cells. This was an about 10 times higher expression level than those of Lag3 and Ikzf4, which are previously known markers for regulatory T cells. In addition, as shown in FIGS. 4 and 5, the expression level of Lrig-1 mRNA was remarkably high in the regulatory T cells compared to other types of immune cells, and in particular, was remarkably high in naturally isolated regulatory T cells (nTreg) compared to induced regulatory T cells (iTreg).

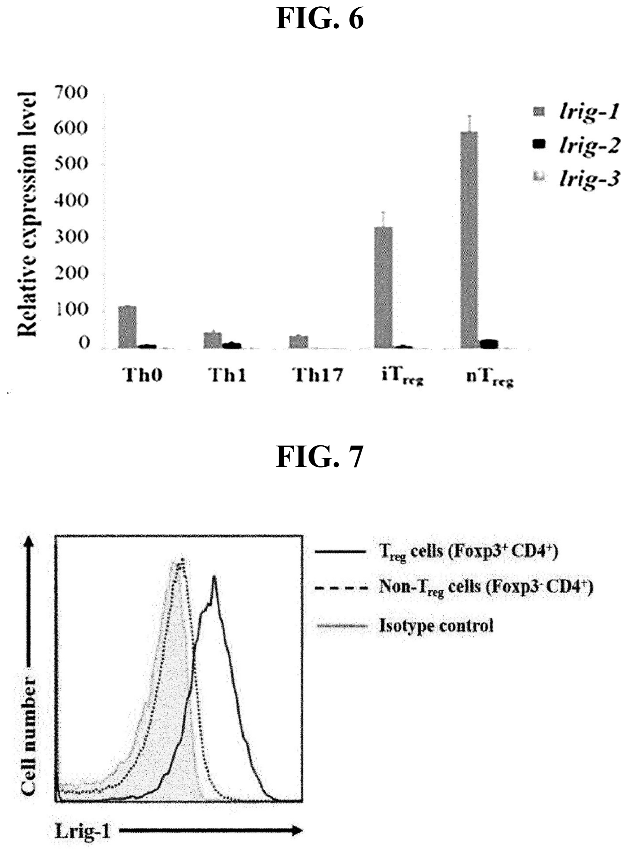

In addition, as shown in FIG. 6, the expression level of Lrig-1 was the highest among the expression levels of Lrig-1, Lrig-2 and Lrig-3 belonging to the Lrig family.

From the above results, it can be seen that the Lrig-1 protein according to the present invention is expressed specifically in regulatory T cells, in particular, naturally occurring regulatory T cells.

### [Example 31 Identification of Specific Expression of Lrig-1 Protein in Regulatory T Cells

It was identified whether the Lrig-1 protein expressed from Lrig-1 mRNA was specifically expressed only in regulatory T cells.

Using FOXP3-RFP-knocked-in mice having red fluorescence protein (RFP) conjugated to the FOXP3 promoter, which is a transcription factor specific for regulatory T cells, CD4⁺ T cells were isolated from the mouse spleens by magnet-activated cell sorting (MACS) using CD4 beads. Subsequently, regulatory T (CD4⁺RFP⁺ T) cells and non-regulatory T (CD4⁺RFP⁻ T) cells were isolated by fluorescence-activated cell sorting (FACS) using RFP protein. The respective cells were stained with the purchased Lrig-1 antibody, a negative control was stained with an isotype antibody, and the expression levels of Lrig-1 therein were measured by fluorescence-activated cell sorting. The results are shown in FIG. 7.

As shown in FIG. 7, the non-regulatory T cells indicated by the dotted line showed almost the same expression level of Lrig-1 as the negative control, whereas there were a large number of cells with a high expression level of Lrig-1 in the regulatory T cells.

From the above results, it can be seen that the Lrig-1 protein according to the present invention is expressed specifically in regulatory T cells.

### [Example 4] Identification of Specific Expression of Lrig-1 Protein on Surface of Regulatory T Cells

In order to be a target of cell therapy, the Lrig-1 protein must be expressed on the surfaces of regulatory T cells, which in turn allows target therapy to be more effectively achieved. Thus, it was identified whether the Lrig-1 protein would be expressed on the surfaces of the regulatory T cells.

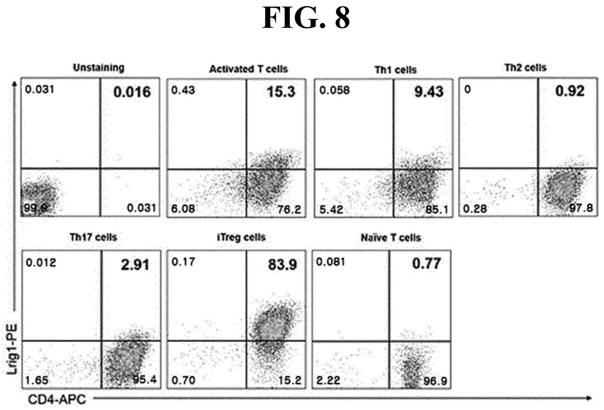

The respective differentiated T cell subsets of Preparation Example 1 were stained with anti-CD4-APC and anti-Lrig-1-PE antibodies, and the expression levels of Lrig-1 on the respective cell surfaces were measured using a fluorescence-activated cell sorter (FACS). The results are illustrated in FIG. 8.

As shown in FIG. 8, Lrig-1 was expressed in an amount of 0.77 to 15.3 in activated T cells, Th1 cells, Th2 cells, Th17 cells, and naive T cells, whereas Lrig-1 was expressed as high as 83.9 in differentiation-induced T cells (iTreg cells).

From the above results, it can be seen that the Lrig-1 protein according to the present invention is not only specifically expressed in regulatory T (Treg) cells, but also is expressed at a higher level, in particular, on the surface of the Treg cells.

### [Preparation Examples 1 to 8] Preparation of Monoclonal Antibodies Specific to Lrig-1 protein

Antibodies specific to the Lrig-1 protein according to the present invention were produced. These antibodies were not produced by specifying a certain epitope, but were produced as antibodies capable of binding to any site on the Lrig-1 protein.

In order to produce the antibodies, cells expressing the Lrig-1 protein were produced. More specifically, a DNA fragment corresponding to SEQ ID NO: 2 and pcDNA (hygro) were cleaved with a cleavage enzyme, incubated at 37°C, and ligated to produce pcDNA into which the DNA sequence of the Lrig-1 protein has been inserted. The produced pcDNA into which SEQ ID NO: 2 has been inserted was introduced through transfection into L cells so that the Lrig-1 protein could be expressed on the surface of the L cells.

Light and heavy chain amino acid sequences capable of binding to Lrig-1 expressed on the cell surface were selected from the Human scFv library, thereby selecting a total of eight heavy and light chains.

The selected heavy and light chain amino acid sequences were fused with the mlgG2a Fc region or the human IgG1 Fc region, thereby producing monoclonal antibodies. The sequences of the monoclonal antibodies are shown in Table 3 below.

**[Table 3]**

| Classification | Clone | Location | Sequence information |
|---|---|---|---|
| Preparation Example 1 | GTC210-01 clone | Heavy chain | SEQ ID NO: 17 |
| | | Heavy chain CDR1 | SEQ ID NO: 46 |
| | | Heavy chain CDR2 | SEQ ID NO: 47 |
| | | Heavy chain CDR3 | SEQ ID NO: 48 |
| | | Light chain | SEQ ID NO: 18 |
| | | Light chain CDR1 | SEQ ID NO: 49 |
| | | Light chain CDR2 | SEQ ID NO: 50 |
| | | Light chain CDR3 | SEQ ID NO: 51 |
| Preparation Example 2 | GTC210-02 clone | Heavy chain | SEQ ID NO: 19 |
| | | Heavy chain CDR1 | SEQ ID NO: 52 |
| | | Heavy chain CDR2 | SEQ ID NO: 53 |
| | | Heavy chain CDR3 | SEQ ID NO: 54 |
| | | Light chain | SEQ ID NO: 20 |
| | | Light chain CDR1 | SEQ ID NO: 55 |
| | | Light chain CDR2 | SEQ ID NO: 56 |
| | | Light chain CDR3 | SEQ ID NO: 57 |
| Preparation Example 3 | GTC210-03 clone | Heavy chain | SEQ ID NO: 21 |
| | | Heavy chain CDR1 | SEQ ID NO: 58 |
| | | Heavy chain CDR2 | SEQ ID NO: 59 |
| | | Heavy chain CDR3 | SEQ ID NO: 60 |
| | | Light chain | SEQ ID NO: 22 |
| | | Light chain CDR1 | SEQ ID NO: 61 |
| | | Light chain CDR2 | SEQ ID NO: 62 |
| | | Light chain CDR3 | SEQ ID NO: 63 |
| Preparation Example 4 | GTC210-04 clone | Heavy chain | SEQ ID NO: 23 |
| | | Heavy chain CDR1 | SEQ ID NO: 64 |
| | | Heavy chain CDR2 | SEQ ID NO: 65 |
| | | Heavy chain CDR3 | SEQ ID NO: 66 |
| | | Light chain | SEQ ID NO: 24 |
| | | Light chain CDR1 | SEQ ID NO: 67 |
| | | Light chain CDR2 | SEQ ID NO: 68 |
| | | Light chain CDR3 | SEQ ID NO: 69 |
| Preparation Example 5 | GTC110-01 clone | Heavy chain | SEQ ID NO: 25 |
| | | Heavy chain CDR1 | SEQ ID NO: 70 |
| | | Heavy chain CDR2 | SEQ ID NO: 71 |
| | | Heavy chain CDR3 | SEQ ID NO: 72 |
| | | Light chain | SEQ ID NO: 26 |
| | | Light chain CDR1 | SEQ ID NO: 73 |
| | | Light chain CDR2 | SEQ ID NO: 74 |
| | | Light chain CDR3 | SEQ ID NO: 75 |
| Preparation Example 6 | GTC110-02 clone | Heavy chain | SEQ ID NO: 27 |
| | | Heavy chain CDR1 | SEQ ID NO: 76 |
| | | Heavy chain CDR2 | SEQ ID NO: 77 |
| | | Heavy chain CDR3 | SEQ ID NO: 78 |
| | | Light chain | SEQ ID NO: 28 |
| | | Light chain CDR1 | SEQ ID NO: 79 |
| | | Light chain CDR2 | SEQ ID NO: 80 |
| | | Light chain CDR3 | SEQ ID NO: 81 |
| Preparation Example 7 | GTC110-03 clone | Heavy chain | SEQ ID NO: 29 |
| | | Heavy chain CDR1 | SEQ ID NO: 82 |
| | | Heavy chain CDR2 | SEQ ID NO: 83 |
| | | Heavy chain CDR3 | SEQ ID NO: 84 |
| | | Light chain | SEQ ID NO: 30 |
| | | Light chain CDR1 | SEQ ID NO: 85 |
| | | Light chain CDR2 | SEQ ID NO: 86 |
| | | Light chain CDR3 | SEQ ID NO: 87 |
| Preparation Example 8 | GTC110-04 mouse antibody | Heavy chain | SEQ ID NO: 31 |
| | | Heavy chain CDR1 | SEQ ID NO: 88 |
| | | Heavy chain CDR2 | SEQ ID NO: 89 |
| | | Heavy chain CDR3 | SEQ ID NO: 90 |
| | | Light chain | SEQ ID NO: 32 |
| | | Light chain CDR1 | SEQ ID NO: 91 |
| | | Light chain CDR2 | SEQ ID NO: 92 |
| | | Light chain CDR3 | SEQ ID NO: 93 |
| Preparation Example 9 | GTC110-04 humanized antibody | Heavy chain | SEQ ID NO: 33 |
| | | Heavy chain CDR1 | SEQ ID NO: 88 |
| | | Heavy chain CDR2 | SEQ ID NO: 89 |
| | | Heavy chain CDR3 | SEQ ID NO: 90 |
| | | Light chain | SEQ ID NO: 34 |
| | | Light chain CDR1 | SEQ ID NO: 91 |
| | | Light chain CDR2 | SEQ ID NO: 92 |
| | | Light chain CDR3 | SEQ ID NO: 93 |

### [Example 5] Evaluation of Binding Capacities of Antibodies According to the Present Invention to Lrig-1 Protein

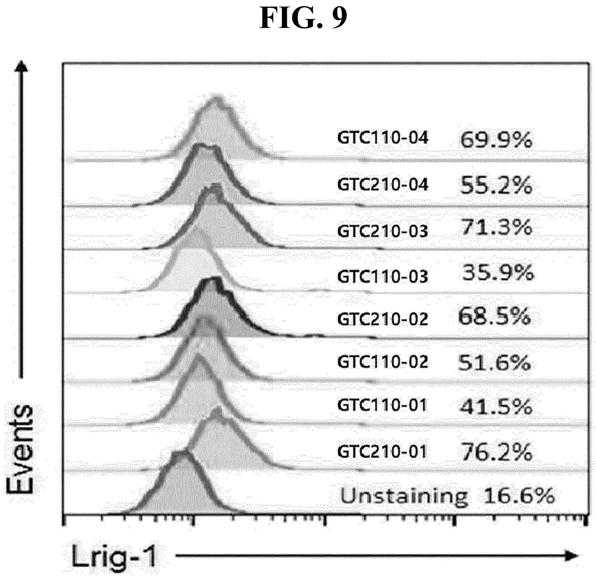

In order to identify whether the monoclonal antibodies according to the present invention, produced in Preparation Examples 1 to 9, well recognize Lrig-1, each of the antibodies of Preparation Examples 1 to 9 was bound to L cells that stably express Lrig-1. Then, an eFlour 670-conjugated secondary antibody capable of recognizing the mouse antibodies was added thereto, and then the binding capacities of the monoclonal antibodies of the Preparation Examples to the Lrig-1 protein were analyzed using FACS. The results are shown in FIG. 9.

As shown in FIG. 9, it could be found that all of the Lrig-1 protein-specific monoclonal antibodies according to the present invention effectively recognized and bound to the Lrig-1 protein present on the surface of L cells.

### [Example 6] Acceptable Changes in CDRs of Antibodies According to the Present Invention

In order to investigate the role of each amino acid position in each CDR, the CDRs of the antibodies of Preparation Example 1 to 9, which bind specifically to the Lrig-1 protein, were listed based on GTC210-03 and analyzed.

Acceptable changes in the CDRs of the antibodies were analyzed. As a result, Table 4 below summarizes acceptable changes in the CDRs of the antibodies that specifically bind to an epitope of Lrig-1 and are effective against immune-related disease by regulating signaling pathways in regulatory T cells, compared to the control, and Table 5 below shows the specific sequences of the CDRs.

**[Table 4] Summary of acceptable changes in CDRs**

| CDR | Position | Acceptable change |
|---|---|---|
| VH CDR1 | S1 | G, N, D |
| | D3 | Y, A |
| VH CDR2 | G1 | L, S, W, A, V |
| | S3 | Y |
| | P4 | H |
| | D5 | G, S |
| | G6 | S, D, |
| | S7 | G |
| | N8 | S |
| | I9 | K, T |
| VH CDR3 | V1 | G, D |
| | G2 | L, I |
| | L3 | G, S |
| | R4 | L, P, N |
| | R6 | K, P, H |
| | Y7 | T, W, L |
| | E8 | G |
| | A9 | L, R, V |
| | S11 | Y |
| | A12 | Y, D, S |
| | Y13 | D, N |
| | G14 | A |
| VL CDR1 | S1 | T |
| | G2 | D |
| | S9 | N |
| | Y11 | N, S, T, D |
| | S13 | T, N, Y |
| VL CDR2 | S1 | D, A |
| | D2 | N |
| | S3 | N |
| | H4 | Q, N |
| VL CDR3 | A1 | G |
| | T2 | S, A |
| | S5 | Y, D |
| | N8 | S |
| | G9 | A |

**[Table 5] Acceptable sequences of CDRs**

| CDR | Sequence | Changed residue | SEQ ID NO. |
|---|---|---|---|
| VH CDR1 | SYDMS | - | 58 |
| | GYDMS | S1→G | 46 |
| | NYYMS | S1→N, D3→Y | 52 |
| | NYDMS | S1→N | 64 |
| | DYDMS | S1→D | 70 |
| | DYYMS | S1→D | 76 |
| | NYAMS | S1→N, D3→A | 82 |
| | NYAMS | S1→N, D3→A | 88 |
| VH CDR2 | GISPDGSNIYYADSVKG | - | 59 |
| | LIYPDSGNKYYADSVK G | G1→L, S3→Y, G6→S, S7→G, I9→K | 47 |
| | GISPGDSSTYYADSVK G | D5→G, G6→D, N8→S, I9→T | 53 |
| | SISPSSGSIYYADSVKG | G1→S, D5→S, G6→S, S7→G, N8→S | 65 |
| | WISHGGGSIYYADSVK G | G1→W, P4→H, D5→G, S7→G, N8→S | 71 |
| | GISHDSGSKYYADSVK G | P4→H, G6→S, S7→G, N8→S, I9→K | 77 |
| | AIYPGGGSIYYADSVK G | G1→A, S3→Y, D5→G, S7→G, N8→S | 83 |
| | VISHGGGSTYYADSVK G | G1→V, P4→H, D5→G, S7→G, N8→S, I9→T | 89 |
| VH CDR3 | VGLRCRYEACSYAYG MDV | - | 60 |
| | GLGLCKTGLCYYYDA MDV | V1→G, G2→L, L3→G, R4→L, R6→K, Y7→T, E8→G, A9→L, S11→Y, A13→Y, Y14→D, G15→A | 72 |
| | DILPCPWGRCYYDYA MDV | V1→D, G2→I, R4→P, Y7→W, E8→G, A9→R, S11→Y, A13→D, G15→A | 84 |
| | VISNCHLGVCYYSNGM DV | G2→I, L3-S, R4→N, R6→H, Y7→L, E8→G, A9→V, S11→Y, A13→S, Y14→N | 90 |
| | HWTTFDY | - | 78 |
| | DAGLSWAGAFDY | - | 48 |
| | GLYSNPNEPFDY | D1→G, A2→L, G3→Y, L4→S, S5→N, W6→P, A7→N, G8→E, A9→P | 54 |
| | DLDAFWRPSFDY | A2→L, G3→D, L4→A, S5→F, A7→R, G8→P, A9→S | 66 |
| VL CDR1 | SGSSSNIGSNYVS | - | 61 |
| | SGSSSNIGSNYVT | S13→T | 49 |
| | TGSSSNIGSNYVS | S1→T | 55 |
| | TGSSSNIGNNNVN | S1→T, S9→N, Y11→N, S13→N | 67 |
| | TGSSSNIGNNSVT | S1→T, S9→N, Y11→S, S13→T | 73 |
| | SGSSSNIGSNNVT | Y11→N, S13→T | 79 |
| | SDSSSNIGSNTVS | G2→D, Y11→T | 85 |
| | SGSSSNIGNNDVY | S9→N, Y11→D, S13--Y | 91 |
| VL CDR2 | SDSHRPS | - | 62 |
| | SDSHRPS | - | 50 |
| | DDSQRPS | S1→D, H4→Q | 56 |
| | SDSHRPS | - | 68 |
| | ADNNRPS | S1→A, S3→N, H4→N | 74 |
| | ANSNRPS | S1→A, D2→N, H4→N | 80 |
| | ADNNRPS | S1→A, S3→N, H4→N | 86 |
| | SDSQRPS | H4→Q | 92 |
| VL CDR3 | ATWDSSLNGYV | - | 63 |
| | GSWDYSLSAYV | A1→G, T2→S, S5→Y, N8→S, G9→A | 51 |
| | GTWDYSLNGYV | A1→G, S5→Y | 57 |
| | GSWDDSLSAYV | A1→G, T2→S, S5→D, N8→S, G9→A | 69 |
| | AAWDSSLSAYV | T2→A, N8→S, G9→A | 75 |
| | GAWDYSLSAYV | A1→G, T2→A, S5→Y, N8→S, G9→A | 81 |
| | GTWDYSLSGYV | A1→G, S5→Y, N8→S | 87 |
| | GTWDYSLSGYV | A1→G, S5→Y, N8→S | 93 |

### [Example 71 Regulation of Signaling Pathways in Regulatory T cells by Antibodies According to the Present Invention

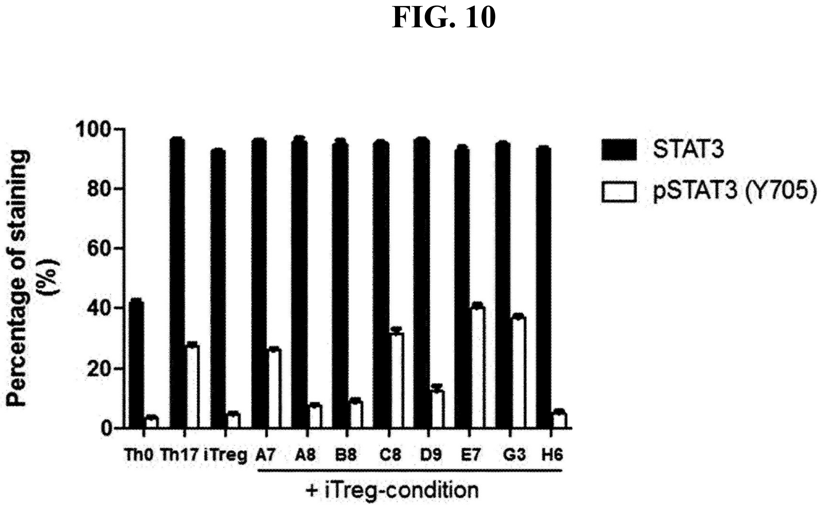

In order to analyze how the monoclonal antibodies of Preparation Examples 1 to 9 according to the present invention affect the signaling pathways in regulatory T cells through the Lrig-1 protein, regulatory T cells were treated with monoclonal antibodies corresponding to Preparation Examples 1 to 9 to stimulate Lrig-1 present on the surface of the regulatory T cells. Then, the level of tyrosine phosphorylation of Stat3 protein present in the stimulated regulatory T cells was analyzed by phosphotyrosine immunoblotting. The results are shown in FIG. 10.

As shown in FIG. 10, it could be found that the Lrig-1 protein-specific monoclonal antibodies (GTC210-01, GTC210-02, GTC210-03 and GTC210-04) according to the present invention increased phosphorylation of Stat3 to the same level as that in Th17 cells. Meanwhile, it could be found that the Lrig-1 protein-specific monoclonal antibodies (GTC110-01, GTC110-02, GTC110-03 and GTC110-04) according to the present invention continued to maintain and decrease the phosphorylation of Stat3 at the same level as that in iTreg cells.

### [Example 8] Identification of Epitope of Lrig-1 Protein

The following experiment was performed to identify a structural epitope, is a site to which the GTC210-03 antibody of Preparation Example 3 can bind, in the extracellular domain of the Lrig-1 protein.

### [8-1] Reagents and Materials

CNBr-activated Sepharose^{™} 4B) (GE, 17-0430-01)
Diethylpyrocarbonate (DEPC, Sigma, 159220)
Imidazole (Acros, 301870010)
Sequencing grade modified trypsin (Promega, V5117)
Chymotrypsin, sequencing grade (Promega, V1062)
Asp-N, sequencing grade (Promega, V1621)
rLyc-C, mass spec grade (Promega, V1671)
Disuccinimidyl dibutyric urea (DSBU; Thermo, A35459)
Dimethyl sulfoxide (DMSO, Sigma, D5879)
Sodium bicarbonate (NaHCO₃, Sigma, 792519)
Sodium chloride (NaCl, Sigma, S3014)
Sodium acetate (NaOAc, Sigma, S2889)
Tris (GE, 17-1321-01), glycine) (GE, 17-1323-01)
ProteaseMAX^{™} surfactant, trypsin enhancer (Promega, V207A)
Acetone (Merck, 1.07021.2521)
Dithiothreitol (DTT; GE, 17-1318-02)
Iodoacetamide (IAA; Sigma, I-6125)
Sodium phosphate monobasic dihydrate (NaH₂PO₄, Sigma, 71505)
Sodium phosphate dibasic hepta-hydrate (Na₂HPO₄, Sigma, S9390)
Syringe filter 0.2-µm (Sartorius Stedim, 16534)
Acetonitrile, methanol, water (HPLC grade, J.T baker)
Formic acid (Sigma, 33015)

### [8-2] Experimental Methods

### [8-2-1] Covalent Labelling MS (CL-MS)

The ratio of antigen and antibody was set to 2:1, and an antigen sample was prepared. Then, DEPC was added thereto so as not to exceed 1%, and then reaction was performed at 37°C for 1 minute and terminated by adding imidazole. Next, a precipitation reaction was performed by adding acetone, and then the supernatant was removed by centrifugation, and the precipitate was well dissolved using 0.1% PM. The dissolved precipitate was degraded by adding trypsin, chymotrypsin, or Asp-N/Lys-C thereto, and then the sugar chains attached to the protein were removed using PNGase-F. Finally, disulfide bonds existing between cysteines of the protein were cleaved using DTT and IAA, and LC-MS and MS2 analysis were performed.

### [8-2-2] Cross-Linking MS (XL-MS)

The ratio of antigen and antibody was set to 2:1, and two antigen samples were prepared. Then, a cross-linker (CSBU) was sufficiently dissolved by DMSO. Thereafter, the sample and DSG were added at a ratio of 1:100, and only DMSO was added to the other sample, and then reaction was performed at 25°C for 1 hour. After the reaction was completed, the protein was degraded using trypsin or chymotrypsin, and then the sugar chains attached to the protein were removed using PNGase-F. Finally, disulfide bonds existing between cysteines of the protein were cleaved using DTT and IAA, and LC-MS and MS2 analysis were performed.

### [8-2-3] LC-MS and MS Analysis Conditions

### 1. Liquid chromatography (LC) conditions

Column: Hypersil GOLD^{™} (1.9 µm, 1 X 100 mm)
Flow rate: 100 µL/min
Mobile phase A: D.W. / 0.2% formic acid
Mobile phase B: ACN / 0.2% formic acid
Temperature): 30°C
LC condition (gradient)

**[Table 7]**

| Step | Total time (min) | Flow rate (µL/min) | A(%) | B(%) |
|---|---|---|---|---|
| 0 | 0.00 | 100.00 | 95.0 | 5.0 |
| 1 | 5.00 | 100.00 | 95.0 | 5.0 |
| 8 | 7.00 | 100.00 | 90.0 | 10.0 |
| 3 | 48.00 | 100.00 | 70.0 | 30.0 |
| 4 | 55.00 | 100.00 | 5.0 | 95.0 |
| 5 | 65.00 | 100.00 | 5.0 | 95.0 |
| 6 | 66.00 | 100.00 | 95.00 | 5.0 |
| 7 | 71.00 | 100.00 | 95.00 | 5.0 |

### 2. MS and MS2 conditions

**[Table 8]**

| HESI source | |
|---|---|
| Sheath gas flow rate | 35 |
| Auxiliary gas flow rate | 10 |
| Sweep gas flow rate | 1 |
| Spray voltage (kV) | 3.5 |
| Spray current (µA) | - |
| Capillary temperature (°C) | 250 |
| S-lens RF level | 50 |
| Auxiliary gas heater temperature (°C) | 200 |

**[Table 9]**

| General | |
|---|---|
| Runtime | From 0 to 71 min |
| Polarity | Positive |
| Default charge state | 2 |

**[Table 10]**

| Full MS | |
|---|---|
| Resolution | 70,000 |
| AGC target | 3.00E+06 |
| Maximum IT | 100 ms |
| Scan range | 200 to 2,000 m/z |

**[Table 11]**

| dd-MS² / dd-SIM | |
|---|---|
| Resolution | 17,500 |
| AGC target | 1.00E+05 |
| Maximum IT | 54 ms |
| Loop count | 10 |
| TopN | 10 |
| Isolation window | 1.6 m/z |
| (N)CE / stepped (N)CE | nce: 27 |

**[Table 12]**

| dd settings | |
|---|---|
| Minimum AGC target | 8.00E+04 |
| Intensity threshold | 1.50E+05 |
| Charge exclusion | Unassigned, 7, 8, >8 |
| Peptide match | Preferred |
| Exclude isotopes | On |
| Dynamic exclusion | 5.0 s |

### [8-3] Covalent labeling MS (CL-MS)

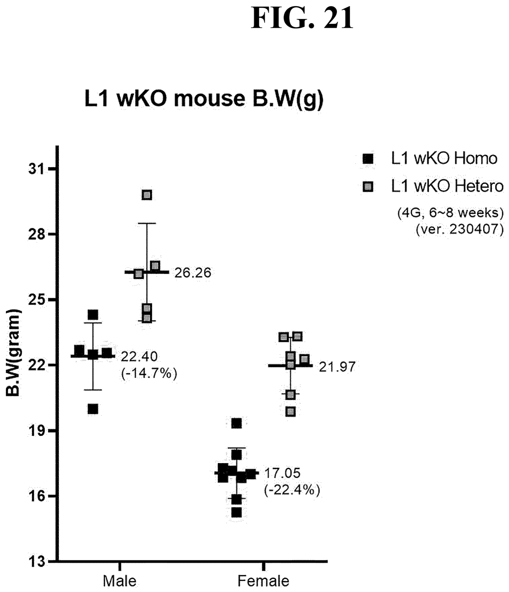

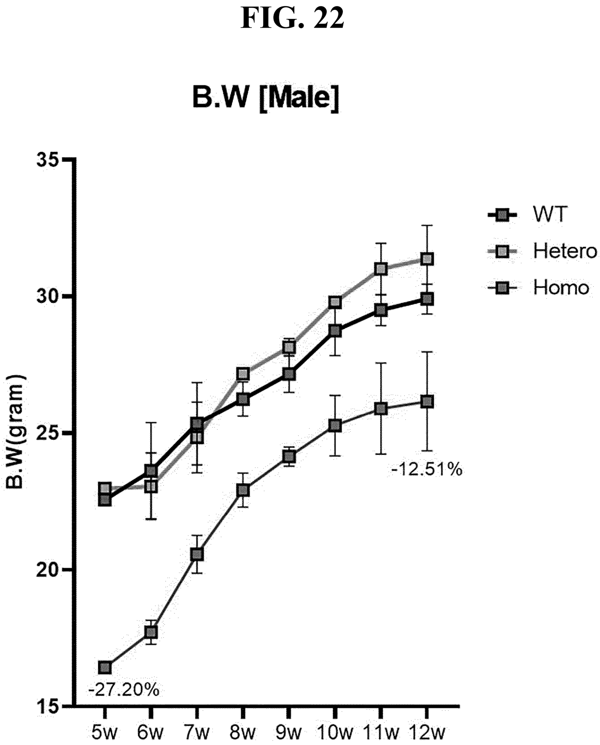

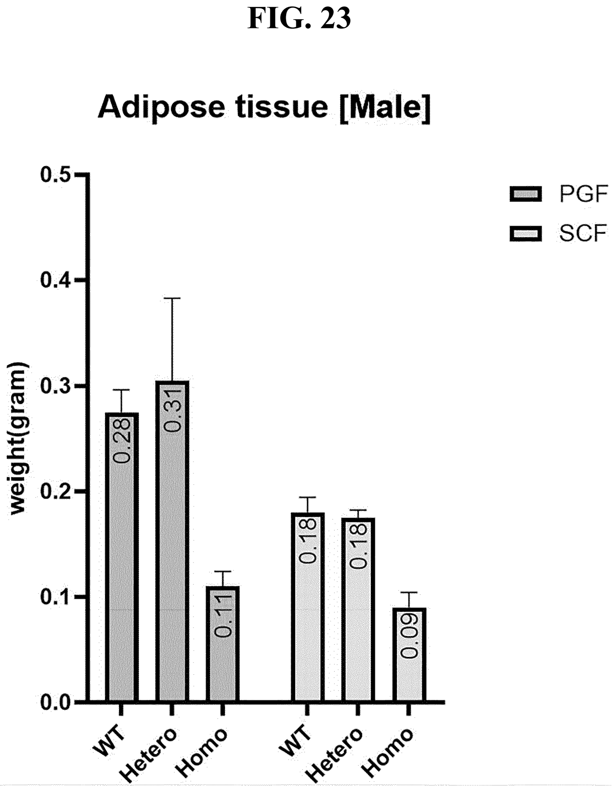

The samples, obtained by treating each of the huTregL1-his (Ag, Control) control sample and the huTregL1-his/E7-mlgG2a (Ag+Ab, Test) test sample with DEPC and then degrading the same by treatment with trypsin, chymotrypsin or Asp-N/Lys-C, were analyzed in triplicate (see FIGS. 21 to 23). The results of MS analysis of the control (Control 1) and the test (Test 1) samples, shown in the analysis results, were matched with the sequence of the control (huTregL1-his) using the BioPhama finder program, and labeled peptides were compared. In addition, the present inventors performed the process of identifying unlabeled and labeled peptides in the control and test samples treated with chymotrypsin, trypsin, or Asp-N/Lys-C. The results of the repeated tests were taken together, and the results obtained by comparing the labeling (%) of the control and test samples (decrease ≥ 5%) were summarized based on the amino acid sequence (Res#) and are shown in FIGS. 21 and 22. Based on the results thus summarized, the sequences were substituted into the huTregL1-his sequence, and then a matching sequence in the results of the two protease tests was found.

As a result, it was found that, among the sequences with a labeling decrease (%) of 5% or more, the sequences E62-L101, D111-L134, and Q542-Y553 had labeling decreases (%) of 13.2%, 15.3%, and 14.7%, respectively, which are more than 10%. In the E62-L101 sequence, the E62-K92 portion (AL) showed a labeling (%) of 65.9%, the L65-F87 portion (Y) showed a labeling (%) of 48.7%, and the L88-L101 portion showed a labeling (%) of 88.6%. These results suggest that, in the E62-L101 sequence, the L88-L101 portion with a labeling (%) of 50% or more is exposed to the outside. It was found that the D111-L134 sequence showed a labeling decrease (%) of 15.3% in the chymotrypsin-treated group, but the RSD of the control was 62.4%. The Q542-Y553 sequence also showed a labeling decrease (%) of 14.7% in the chymotrypsin-treated group, and the labeling (%) of the control antigen was 50% or more, indicating that it is an externally exposed portion of the protein structure.

It was finally determined that the present sequences derived from the CL-MS analysis results for the test sample (huTregL1-his/E7-mlgG2a) showed a labeling decrease (%) of 5% or more in two or more protease-treated groups, and that the E62-L101 sequence, which is a sequence determined to be exposed to the outside, corresponds to an epitope site.

### [8-4] Cross-linking MS (XL-MS)

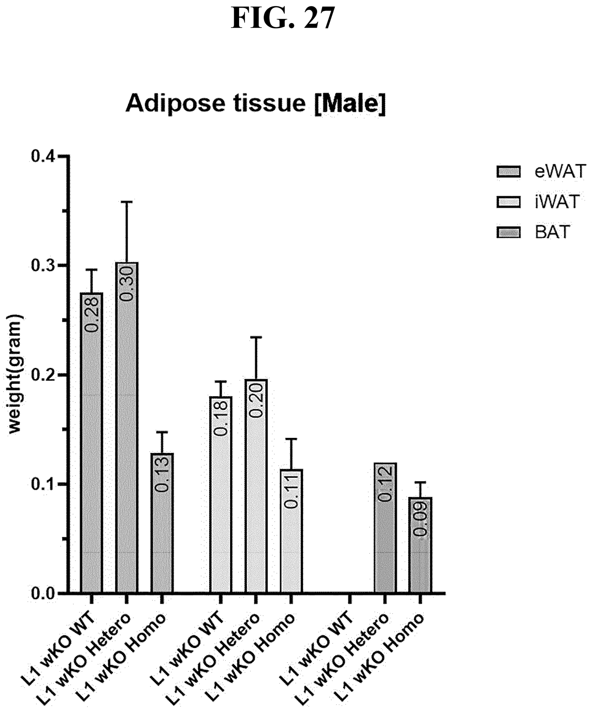

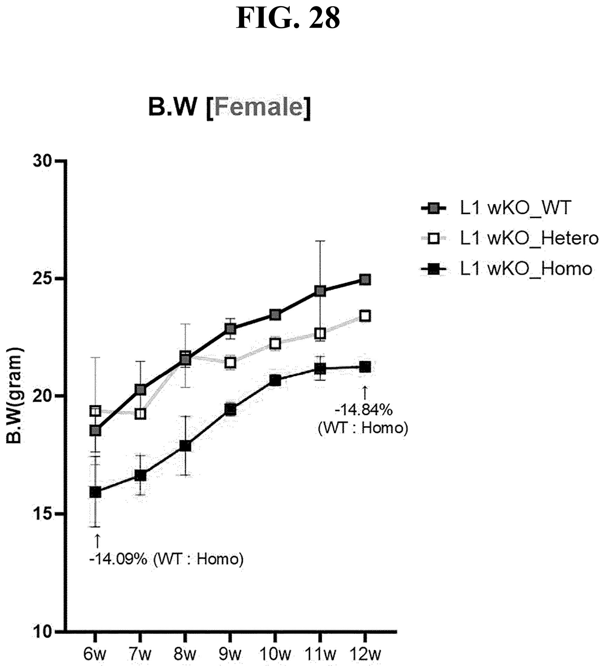

A test (huTregL1-his/E7-mlgG2a) mixture sample was prepared, treated with disuccinimidyl dibutyric urea (DSBU), a cross-linker, and degraded with protease (chymotrypsin or trypsin) (FIGS. 27 and 28). Thereafter, peptides obtained by degradation with chymotrypsin and trypsin were compared with the control, and peptide sequences that were not detected (including 2% or less) in the test tube were identified. In addition, in these peptides, the form (mono) to which only the cross-linker bound was examined and reconfirmed (M%), and finally, the peptide sequences not detected in the test samples were selected and determined to be epitope sites. For three sequences, including L88-K92, S90-L101, and N211-W222, the percentage (T%) of peptides of the same sequence detected in the test sample compared to the control group was found to be 2% or less in either chymotrypsin or trypsin. T158-F174 and G442-K476 were reduced in chymotrypsin (Y) and trypsin (T), respectively. The only sequence in which T% was not detected (0%) was S362-K365 in the trypsin (T)-treated group. Through cross-linking MS analysis, three sequences, L88-K92, S90-L101, and N211-W222, which were significantly reduced in the two protease-treated groups, were determined to be epitope sites.

### [8-5] Conclusion

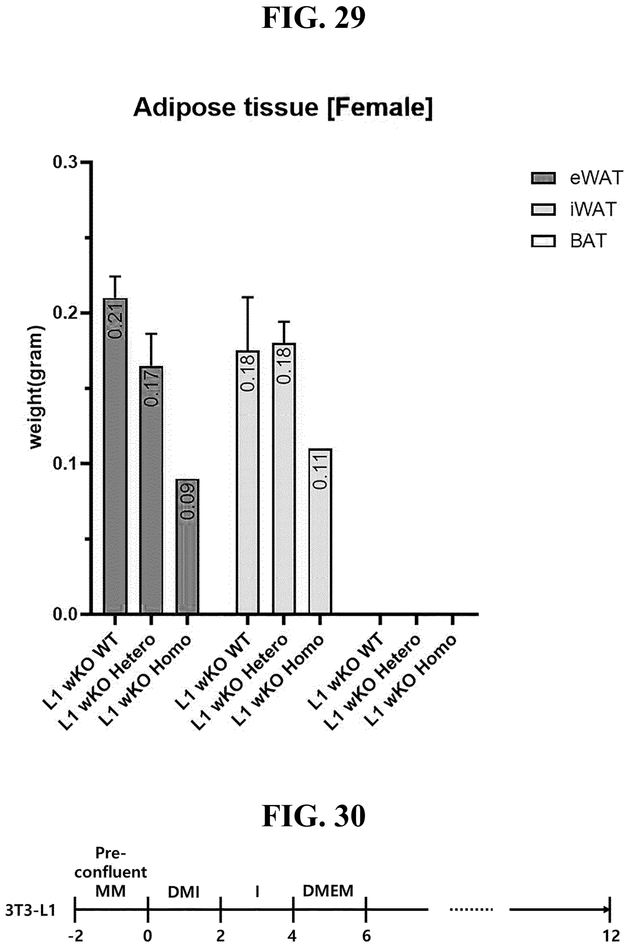

As shown in FIGS. 29 and 30, the epitope was predicted by taking the above results together. Specifically, two sequences, including L65-L101 and G452-K476 of huTregL1-his (Ag), were identified to be matching sequences in CL-MS and XL-MS analysis results. The L88-K92 portion in L65-L101 showed a labeling decrease (%) in the two protease-treated groups (chymotrypsin, and Asp-N/Lys-C) as a result of CL-MS, and was also significantly reduced in the two protease-treated groups (chymotrypsin, and trypsin) as a result of XL-MS analysis. The S95-L101 portion was an epitope identified in both chymotrypsin and trypsin in XL-MS. In addition, the results of CL-MS showed that the L88-L101 portion with a labeling (%) of 50% or more was exposed to the outside. In particular, the amino acid sequence of L88-L101 was composed of consecutive hydrophilic amino acids (Q89-S95).

In addition, in the case of G452-K476, the A472-F480 portion was found to be exposed to the outside as a result of CL-MS analysis, and in particular, the A472-K476 portion was determined to correspond to an epitope. Although N211-W222 was selected as an epitope site in XL-MS, it did not match with that in the CL-MS analysis, and furthermore, this sequence portion was had a labeling (%) of 50% or less, indicating that it was located internally in the structure. Thus, it was excluded from the epitope.

### [Example 9] Confirming the therapeutic effect of Claims specifically binding to Lrig-1 on metabolic diseases - Analysis of experimental results in TregL1 knockout mice

### [9-1] Preparation of L1 wKO (Lrig-1 CreERT2) mice

L1 wKO(Lrig-1 CreERT2) mice are Lrig1 whole-body knockout mice, in which Lrig1 is knocked out by inserting a creERT2 sequence into the translational initiation site of the endogenous Lrig1 locus, and the expression of Lrig1 can be reported as creERT2.

### [9-2] Observation of weight changes in L1 wKO (Lrig-1 CreERT2) mice

The L1 wKO(Lrig-1 CreERT2) mice prepared in Example 9-1 were divided into homo, hetero knockout mice, and male and female mice and observed for weight changes for 6-8 weeks.

The genotypes and weight changes of L1 wKO(Lrig-1 CreERT2) mice are shown in Table 12 and Figure 21 below.

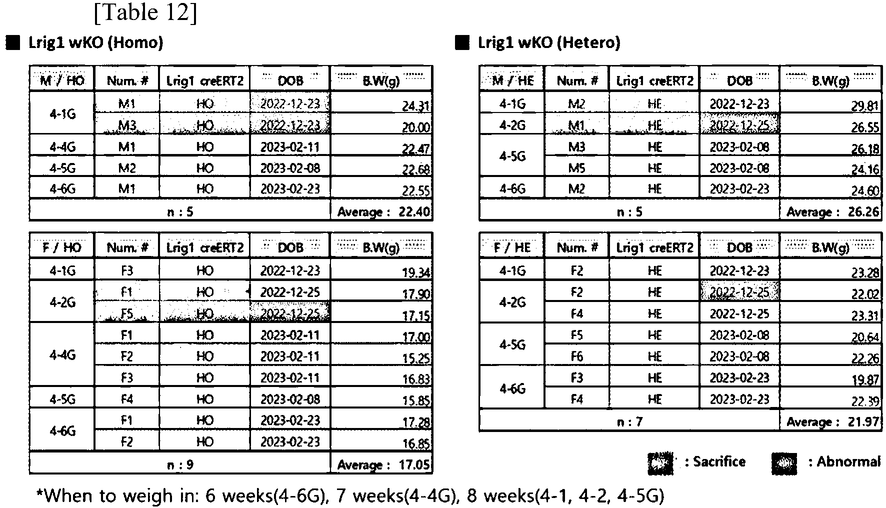

The results showed that among the L1 wKO(Lrig-1 CreERT2) mice, the homo knockout mice weighed 14.7% to 22.4% less than the hetero knockout mice.

### [9-2] Confirmation of reduced adipose tissue and reduced body weight in L1 wKO(Lrig-1 CreERT2) mice

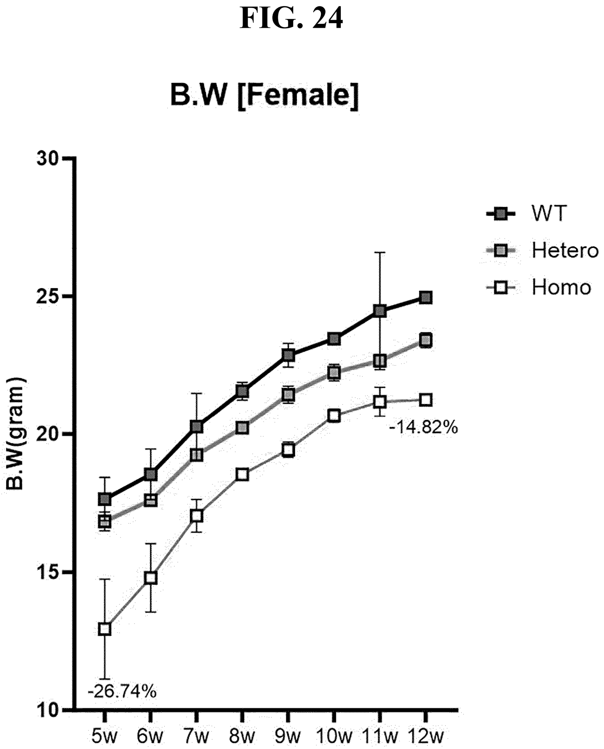

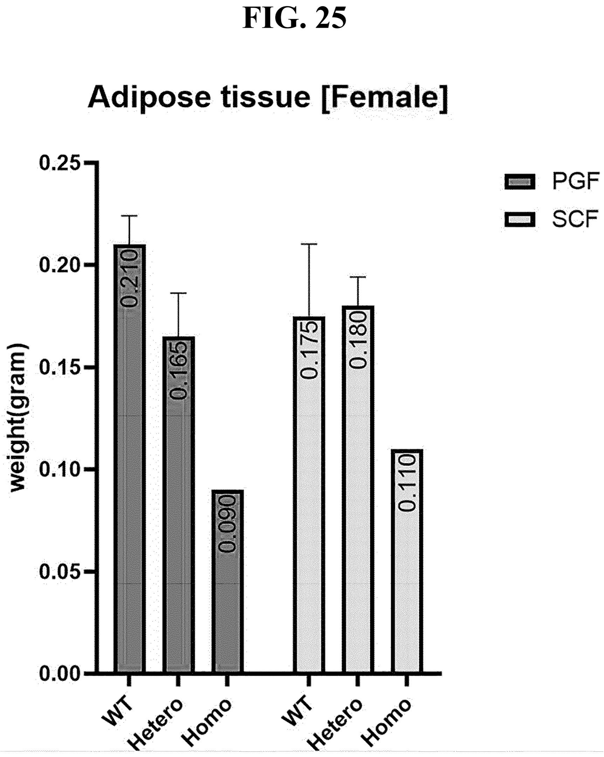

We confirmed the changes in adipose tissue weight and body weight of L1 wKO(Lrig-1 CreERT2) mice on two occasions. Specifically, we first checked the adipose tissue and body weight changes in male and female mice for 5 to 12 weeks, which are shown in FIGS. 22 to 25.

As a result, in the Homo knockout mice, the body weight of males decreased by 12.51% compared to the control group at week 12, and visceral fat and pia fat also decreased, and in females, the body weight of males decreased by 14.82% compared to the control group at week 12, and visceral fat and subcutaneous fat also decreased.

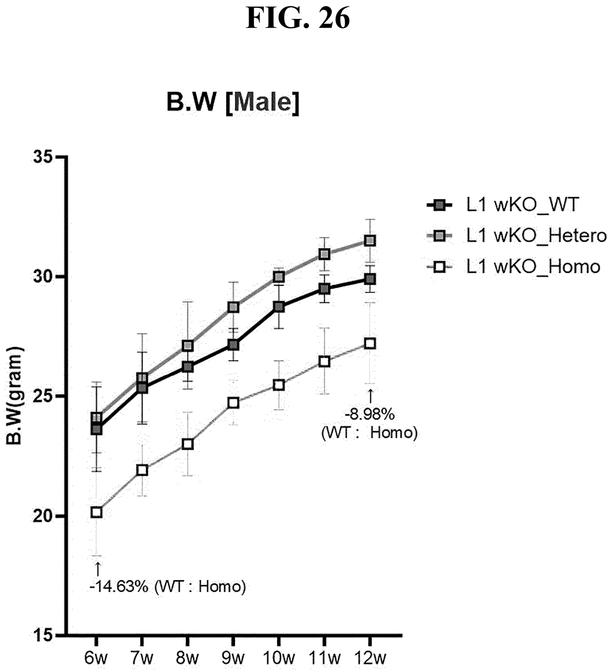

Secondly, we again checked the adipose tissue and body weight changes of male and female mice from 6 to 12 weeks, which are shown in Figures 26 to 29.

The results showed that the body weight of males in the homo knockout mice was reduced by 8.98% at week 12 compared to the control group, and visceral and pia fat were also reduced, especially brown adipocytes. In females, body weight was reduced by 14.84% compared to the control group at week 12, and visceral and subcutaneous fat were also reduced.

Thus, inhibiting the function of TregL1 reduces adipocytes and weight, thus confirming that it has therapeutic effects on metabolic diseases including obesity.

### [Example 10] Diagnosis of metabolic diseases by identifying increased Lrig-1 in adipocytes

### [10-1] Differentiation of 3T3 cells into adipocytes

The 3T3-L1 cell line, a cell line prior to differentiation into adipocytes and expressing Lrig-1, was seeded at 1x105/well in 24-well plates and cultured in DMI medium for 2 days, followed by insulin medium for 2 days, and DMEM medium for 2 days.

The specific experimental protocol is shown in Table 13 and Figure 30 below.

**[Table 13]**

| | **Reagents** | **Conc.** | **Cat. No.** |
|---|---|---|---|
| **Completed DMEM (DMEM)** | **Dulbecco's modified Eagle's medium (DMEM)** | | **Cytiva, SH30243.01** |
| | **Fetal bovine serum** | **10%** | **Cytiva, SV30207.02** |
| | **Penicillin-Streptomycin** | **1%** | **Thermo Fisher, 15140122** |
| **Maintain media (MM)** | **Dulbecco's modified Eagle's medium (DMEM)** | | **Cytiva, SH30243.01** |
| | **Bovine Calf serum** | **10%** | **Cytiva, SH30073.03H1** |
| | **Penicillin-Streptomycin** | **1%** | **Thermo Fisher, 15140122** |
| **DMI** | **Dulbecco's modified Eagle's medium (DMEM)** | | **Cytiva, SH30243.01** |
| | **Fetal bovine serum** | **10%** | **Cytiva, SV30207.02** |
| | **Penicillin-Streptomycin** | **1%** | **Thermo Fisher, 15140122** |
| | **Dexamethasone (Dex)** | **1 µM** | **Sigma, D4902** |
| | **3-isobutyl-1-methylxanthine (IBMX)** | **520 µM** | **Sigma, 228420010** |
| | **Insulin (I)** | **1 µM** | **Sigma, I5500** |
| **I** | **Dulbecco's modified Eagle's medium (DMEM)** | | **Cytiva, SH30243.01** |
| | **Fetal bovine serum** | **10%** | **Cytiva, SV30207.02** |
| | **Penicillin-Streptomycin** | **1%** | **Thermo Fisher, 15140122** |
| | **Insulin (I)** | **167nM** | **Sigma, 15500** |
| **FACS analysis** | **Mouse LRIG1 Alexa488-conjugated Antibody** | | **R&D systems, FA83688G** |

### [10-2] Analysis of Lrig-1 expression in differentiated adipocytes.

In this study, the degree of Lrig-1 expression in differentiated adipocytes was analyzed by flow cytometry using a mouse Lrig-1 Alexa488-conjugated Claim.

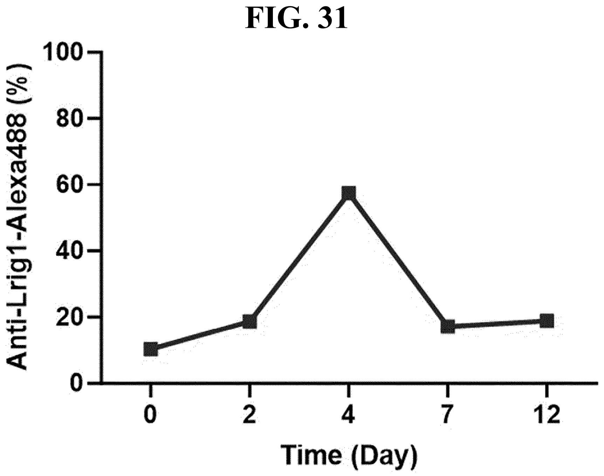

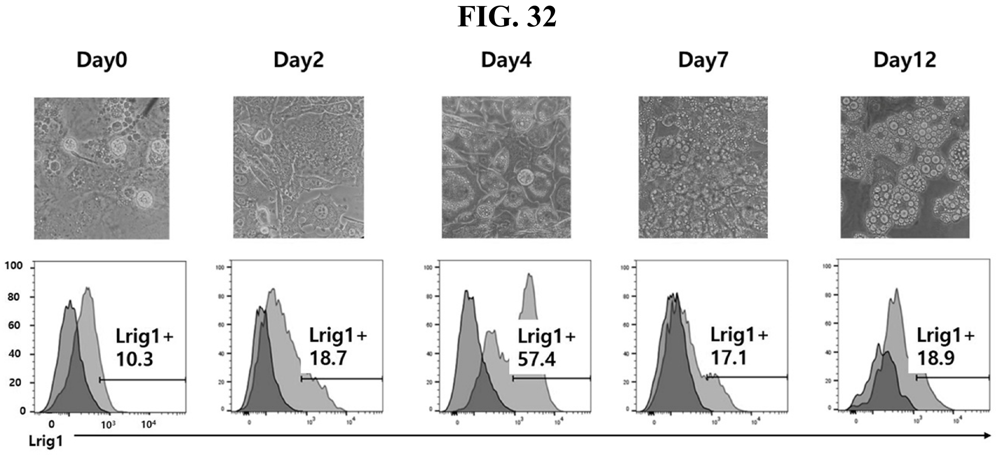

The results are shown in FIGS. 31 and 32, and it can be seen that the expression of Lrig-1 is highest on day 4, which can be used to diagnose metabolic diseases including obesity.

Although the present invention has been described in detail above, the scope of the present invention is not limited thereto. It will be obvious to those skilled in the art that various modifications and changes can be made without departing from the technical spirit of the present invention as described in the claims.

## Claims

**1.** A diagnostic composition for the diagnosis of a metabolic disease, comprising an agent for measuring the expression level of a Lrig-1 protein.

**2.** The diagnostic composition of Claim 1,
wherein the Lrig-1 protein is present on the surface of an adipocyte.

**3.** The diagnostic composition of Claim 2,
wherein an agent for measuring the expression level of a Lrig-1 protein comprises at least one selected from the group consisting of a primer, probe, LNA, and antisense nucleotides that specifically bind to the gene.

**4.** The diagnostic composition of Claim 2,
wherein an agent for measuring the expression level of a Lrig-1 protein is an antibody or antigen-binding fragment that specifically binds to Lrig-1.

**5.** The diagnostic composition of Claim 4,
wherein the antibody or antigen-binding fragment specifically binds to at least one epitope selected from the group consisting of amino acid sequences represented by SEQ ID NO: 35 to SEQ ID NO: 45.

**6.** The diagnostic composition of Claim 5,
wherein the antibody is a chimeric antibody, a humanized antibody, a bivalent-bispecific molecule, a minibody, a domain antibody, a bispecific antibody, an antibody mimic, a diabody, a triabody, a tetrabody, or a fragment thereof.

**7.** The diagnostic composition of Claims 1 to 6,
wherein the metabolic disease is at least one selected from the group consisting of insulin resistance disease, obesity, diabetes, dyslipidemia, liver disease, kidney damage, arteriosclerosis, and hypertension.

**8.** A kit for diagnosing a metabolic disease comprising the diagnostic composition of any of Claims 1 to 7.

**9.** The kit of Claim 8,
wherein the metabolic disease is at least one selected from the group consisting of insulin resistance disease, obesity, diabetes, dyslipidemia, liver disease, kidney damage, arteriosclerosis, and hypertension.

**10.** A method of providing information for diagnosing a metabolic disease, comprising the step of measuring a level of Lrig-1 protein expression in a biological sample isolated from a subject.

**11.** The method of Claim 10,
wherein the Lrig-1 protein is present on the surface of an adipocyte.

**12.** The method of Claim 11,
wherein the expression level of the Lrig-1 protein measured in a biological sample is higher or lower than a normal control, predicting that the subject has a metabolic disease.

**13.** The method of Claim 12,
wherein the metabolic disease is at least one selected from the group consisting of insulin resistance disease, obesity, diabetes, dyslipidemia, liver disease, kidney damage, arteriosclerosis, and hypertension.

**14.** A diagnostic device for a metabolic disease comprises a measurement portion for measuring an expression level of Lrig-1 in a biological sample obtained from a subject; and a detection part for outputting, from the expression level of Lrig-1 measured in said measurement part, whether the intended individual has developed or is likely to develop a bone disease.

**15.** The diagnostic device of Claim 14,
wherein the metabolic disease is at least one selected from the group consisting of insulin resistance disease, obesity, diabetes, dyslipidemia, liver disease, kidney damage, arteriosclerosis, and hypertension.

**16.** A pharmaceutical composition for preventing or treating metabolic disease comprising, as an active ingredient, antibody or antigen-binding fragment that specifically binds to Lrig-1.

**17.** A pharmaceutical composition for preventing or treating metabolic disease comprising, as an active ingredient, a binding molecule which binds specifically to an epitope comprising at least one polypeptide selected from the group consisting of polypeptides consisting of the amino acid sequences represented by SEQ ID NO: 35 to SEQ ID NO: 45.

**18.** The pharmaceutical composition of Claim 17,
wherein the binding molecule is an antibody or an antigen-binding fragment.

**19.** The pharmaceutical composition of Claim 18,
wherein the antibody is a chimeric antibody, a humanized antibody, a bivalent-bispecific molecule, a minibody, a domain antibody, a bispecific antibody, an antibody mimic, a diabody, a triabody, a tetrabody, or a fragment thereof.

**20.** A pharmaceutical composition for preventing or treating metabolic disease comprising, as an active ingredient, a chimeric antigen receptor (CAR) comprising an antigen-specific binding domain, a linking domain, and a CD3 zeta (ζ) signaling domain,
wherein the antigen-specific binding domain is a fusion protein comprising: an antigen-specific binding domain that binds specifically to an epitope comprising at least any one polypeptide selected from the group consisting of polypeptides consisting of the amino acid sequences represented by SEQ ID NO: 35 to SEQ ID NO: 45; and an immunoglobulin Fc region.

**21.** A pharmaceutical composition for preventing or treating metabolic disease comprising, as an active ingredient, an antibody-drug conjugate comprising: a binding molecule; and a drug,
wherein the binding molecule which binds specifically to an epitope comprising at least one polypeptide selected from the group consisting of polypeptides consisting of the amino acid sequences represented by SEQ ID NO: 35 to SEQ ID NO: 45.

**22.** The pharmaceutical composition of Claim 21,
wherein the binding molecule is an antibody or an antigen-binding fragment.

**23.** The pharmaceutical composition of Claims 16 to 23,
wherein the antibody is a chimeric antibody, a humanized antibody, a bivalent-bispecific molecule, a minibody, a domain antibody, a bispecific antibody, an antibody mimic, a diabody, a triabody, a tetrabody, or a fragment thereof.

**25.** A pharmaceutical composition for the prevention or treatment of a metabolic disease, comprising as an active ingredient a compound that binds to Lrig-1 and inhibits the function of Lrig-1.

**26.** The pharmaceutical composition of Claim 25,
wherein the compound inhibits the function of Lrig-1 through antagonism of Lrig-1.

**27.** The pharmaceutical composition of Claim 26,
wherein the Lrig-1 protein is present on the surface of an adipocyte or on the surface of a regulatory T cell.

**28.** The pharmaceutical composition of Claim 27,
wherein the compound is an Lrig-1 inhibitor.

**29.** A method of preventing or treating a metabolic disease, comprising administering to an individual an antibody or antigen-binding fragment that specifically binds to Lrig-1 as an active ingredient.

**30.** A method of preventing or treating a metabolic disease comprising administering to an individual, as an active ingredient, a binding molecule that specifically binds to at least one epitope selected from the group consisting of amino acid sequences represented by SEQ ID NO: 35 through SEQ ID NO: 45.

**31.** A method of preventing or treating a metabolic disease comprising administering to an individual, as an active ingredient, a chimeric antigen receptor (CAR) comprising an antigen-specific binding domain, a linking domain, and a CD3 zeta (ζ) signaling domain,
wherein the antigen-specific binding domain is a fusion protein comprising: an antigen-specific binding domain that binds specifically to an epitope comprising at least any one polypeptide selected from the group consisting of polypeptides consisting of the amino acid sequences represented by SEQ ID NO: 35 to SEQ ID NO: 45; and an immunoglobulin Fc region.

**32.** A method of preventing or treating a metabolic disease comprising administering to an individual, as an active ingredient, a binding molecule which binds specifically to an epitope comprising at least one polypeptide selected from the group consisting of polypeptides consisting of the amino acid sequences represented by SEQ ID NO: 35 to SEQ ID NO: 45.

**33.** A method of preventing or treating a metabolic disease comprising administering to an individual, as an active ingredient, a compound that binds to Lrig-1 and inhibits the function of Lrig-1.
